(19) **Europäisches Patentamt / European Patent Office / Office européen des brevets**

(11) **EP 4 076 240 B1**

(12) **EUROPEAN PATENT SPECIFICATION**

(45) Date of publication and mention
of the grant of the patent:
**01.04.2026 Bulletin 2026/14**

(21) Application number: **20903305.9**

(22) Date of filing: **18.12.2020**

(51) International Patent Classification (IPC):
*A61B 18/00* (2006.01)     *A61B 18/12* (2006.01)
*A61B 18/14* (2006.01)     *A61B 18/16* (2006.01)
*A61B 90/00* (2016.01)

(52) Cooperative Patent Classification (CPC):
**A61B 18/1492; A61B 18/1206;** A61B 18/1233;
A61B 2018/00029; A61B 2018/00172;
A61B 2018/00375; A61B 2018/00577;
A61B 2018/00613; A61B 2018/00648;
A61B 2018/00654; A61B 2018/00666;
A61B 2018/00702; A61B 2018/00708;
A61B 2018/00744; A61B 2018/00791;     (Cont.)

(86) International application number:
**PCT/US2020/066205**

(87) International publication number:
**WO 2021/127558 (24.06.2021 Gazette 2021/25)**

(54) **TREATMENT OF CARDIAC TISSUE WITH PULSED ELECTRIC FIELDS**

BEHANDLUNG VON HERZGEWEBE MIT GEPULSTEN ELEKTRISCHEN FELDERN

TRAITEMENT DE TISSU CARDIAQUE À L'AIDE DE CHAMPS ÉLECTRIQUES PULSÉS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.12.2019   US 201962949633 P
26.03.2020   US 202063000275 P
25.09.2020   US 202063083644 P**

(43) Date of publication of application:
**26.10.2022   Bulletin 2022/43**

(73) Proprietor: **Cardiofocus, Inc.
Marlborough, MA 01752 (US)**

(72) Inventors:
• **CASTELLVI, Quim**
**08750 Barcelona (ES)**
• **GUNDERT, Timothy, James**
**Discovery Bay, CA 94505 (US)**
• **NEAL, Robert, E., II**
**Redwood City, California 94061 (US)**
• **WALDSTREICHER, Jonathan, R.**
**Orange, New Jersey 07052 (US)**

• **GANDIONCO, Isidro**
**Fremont, California 94536 (US)**
• **GIROUARD, Steven, D.**
**Chagrin Falls, Ohio 44022 (US)**
• **MEDIRATTA, Vikramaditya**
**Scottsdale, Arizona 85255 (US)**
• **TAYLOR, Kevin, James**
**San Mateo, California 94403 (US)**
• **VACHANI, Armaan, G.**
**Foster City, California 94404 (US)**
• **KRIMSKY, William, S.**
**Forest Hill, Maryland 21050 (US)**
• **PENDEKANTI, Rajesh**
**Chino Hills, California 91709 (US)**
• **EYSTER, Curt, Robert**
**Rancho Cucamonga, California 91701 (US)**

(74) Representative: **Studio Torta S.p.A.
Via Viotti, 9
10121 Torino (IT)**

(56) References cited:
**WO-A1-2018/208795     WO-A1-2019/055512
WO-A1-2019/217317     US-A- 6 012 457**

EP 4 076 240 B1

US-A1- 2014 206 987     US-B2- 8 641 704

(52) Cooperative Patent Classification (CPC): (Cont.)
A61B 2018/00827; A61B 2018/00839;
A61B 2018/00875; A61B 2018/00898;
A61B 2018/1253; A61B 2018/126; A61B 2018/128;
A61B 2018/1293; A61B 2018/167; A61B 2090/065;
A61B 2217/007; A61B 2218/002; A61B 2562/0247;
A61B 2562/0257; A61B 2562/0271

**Description**

BACKGROUND

Therapeutic energy can be applied to the heart and vasculature for the treatment of a variety of conditions, including atherosclerosis (particularly in the prevention of restenosis following angioplasty) and arrythmias, such as atrial fibrillation. Atrial fibrillation is the most common sustained cardiac arrhythmia, and severely increases the risk of mortality in affected patients, particularly by causing stroke. In this phenomenon, the heart is taken out of normal sinus rhythm due to the production of erroneous electrical impulses. Atrial fibrillation is thought to be initiated in the myocardial sleeves of the pulmonary veins (PVs) due to the presence of automaticity in cells within the myocardial tissue of the PVs. Pacemaker activity from these cells is thought to result in the formation of ectopic beats that initiate atrial fibrillation. PVs are also thought to be important in the maintenance of atrial fibrillation because the chaotic architecture and electrophysiological properties of these vessels provides an environment where atrial fibrillation can be perpetuated. Thus, destruction or removal of these aberrant pacemaker cells within the myocardial sleeves of the PVs has been a goal and atrial fibrillation is often treated by delivering therapeutic energy to the pulmonary veins. However, due to reports of PV stenosis, the approach has been conventionally modified to one that targets PV antra to achieve conduction block between the PVs and the left atrium. The PV antra encompass, in addition to the pulmonary veins, the left atrial roof and posterior wall and, in the case of the right pulmonary vein antra, a portion of the interatrial septum. In some instances, this technique offers a higher success rate and a lower complication rate compared with pulmonary vein ostial isolation.

Thermal ablation therapies, especially radiofrequency (RF) ablation, are currently the "gold standard" to treat symptomatic atrial fibrillation by localized tissue necrosis. Typically, RF ablation is used to create a ring of ablation lesions around the outside of the ostium of each of the four pulmonary veins. RF current causes desiccation of tissue by creating a localized area of heat that results in discrete coagulation necrosis. The necrosed tissue acts as a conduction block thereby electrically isolating the veins.

Despite the improvements in reestablishing sinus rhythm using available methods, both success rate and safety are limited. RF ablation continues to present multiple limitations including long procedure times to perform pulmonary vein isolation with RF focal catheters, potential gaps in ablation patterns due to point-by-point ablation technique with conventional RF catheters, difficulty in creating and confirming transmural ablation lesions, char and/or gas formation at the catheter tip-tissue interface due to high temperatures, which may lead to thrombus or emboli during ablation, and thermal damage to collateral extracardiac structures, which include pulmonary vein stenosis, phrenic nerve injury, esophageal injury, atrio-esophageal fistula, peri-esophageal vagal injury, perforations, thromboembolic events, vascular complications, and acute coronary artery occlusion, to name a few. These limitations are primarily attributed to the continuous battle clinicians have faced balancing effective therapeutic dose with inappropriate energy delivery to extracardiac tissue.

Thus, while keeping the technique in clinical practice, safer and more versatile methods of removing abnormal tissue have been used, including irreversible electroporation (IRE), a non-thermal therapy based on the unrecoverable permeabilization of cell membranes caused by particular short pulses of high voltage energy. IRE has been found to be tissue-specific, triggering apoptosis rather than necrosis, and safer for the structures adjacent the myocardium. However, thus far, the success of these IRE methodologies has been heterogeneous. In some instances, the delivery of IRE energy has resulted in incomplete block of the aberrant electrical rhythms. This may be due to a variety of factors, such as irregularity of treatment circumferentially around the pulmonary veins, lack of transmural delivery of energy or other deficiencies in the delivery of energy. In either case, atrial fibrillation is not sufficiently treated or atrial fibrillation recurs at a later time. Therefore, improvements in atrial fibrillation treatment are desired. Such treatments should be safe, effective, and lead to reduced complications. At least some of these objectives will be met by the systems, devices and methods described herein.

SUMMARY OF THE INVENTION

Described herein are embodiments of apparatuses, systems and methods for treating target tissue, particularly cardiac tissue. The invention is defined by independent claim 1. Further embodiments are described in independent claims 2-14. All references to methods below are not part of the claimed invention as such, but are useful for the general understanding of the invention .

BRIEF DESCRIPTION OF THE DRAWINGS

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document.

Fig. 1 illustrates an embodiment of a tissue modification system.

Figs. 2A-2B illustrates embodiments of a treatment catheter configured to deliver focal therapy.

Fig. 3 illustrates a portion of the heart showing a cut-away of the right atrium and left atrium with a treatment catheter positioned therein.

Fig. 4 illustrates the repeated application of energy in point by point fashion around the left inferior pulmonary vein with the use of the treatment catheter to create a circular treatment zone.

Fig. 5 illustrates an embodiment of a waveform of a signal prescribed by an energy delivery algorithm.

Fig. 6 illustrates an example waveform prescribed by an energy delivery algorithm wherein the waveform has voltage imbalance.

Fig. 7 illustrates further examples of waveforms having unequal voltages.

Fig. 8 illustrates examples of waveforms having unequal pulse widths.

Fig. 9 illustrates an example waveform prescribed by another energy delivery algorithm wherein the waveform is monophasic, a special case of imbalance whereby there is only a positive or only a negative portion of the waveform.

Fig. 10 illustrates further examples of waveforms having monophasic pulses.

Fig. 11 illustrates examples of waveforms having phase imbalances.

Fig. 12 illustrates an example waveform prescribed by another energy delivery algorithm wherein the pulses are sinusoidal in shape rather than square.

Fig. 13 illustrates an embodiment of a conventional ablation catheter.

Fig. 14A illustrates high voltage energy delivery through a conventional ablation catheter having a plurality of electrodes.

Fig. 14B illustrates a cross-section of shaft of Fig. 14A showing the insulated conduction wires corresponding the electrodes.

Fig. 15 schematically illustrates resistors positioned to steer the energy through the conduction wires in a pre-determined fashion so that the voltage differentials stay below a particular threshold level.

Figs. 16A-16B illustrate an increase in threshold for arcing when using a resistor network as described herein.

Figs. 17A-17C illustrate the use of the resistor network and systems described herein to shape the electric field delivered by the catheter.

Fig. 18 provides a schematic illustration of a cross-section of a lumen of a pulmonary vein surrounded by cardiac tissue along with an electrode illustrated as contacting the cardiac tissue via the lumen.

Fig. 19 is a graph illustrating the association between energy and treatment area depth when energy is delivered according to the methods illustrated in Fig. 18.

Figs. 20-21 illustrate by mathematical modeling the current distribution of PEF energy emanating from a delivery electrode of a catheter under different conditions.

Fig. 22 provides a graphical illustration of current density vs. penetration depth in homogenous vs. non-homogenous tissue.

Figs. 23A-23B illustrate the effect of contact force on lesion size, in particular lesion width and depth.

Fig. 24 illustrates a thermal profile for a catheter electrode.

Fig. 25 illustrates a treatment catheter having thermal sensing and irrigation.

Fig. 26 illustrates an example setup wherein an interface connector is utilized with an embodiment of a tissue modification system.

Figs. 27-28 illustrate an embodiment of the interface connector.

Fig. 29 illustrates an outcome of "AND" logic of the generator footswitch signal and the R-wave trigger signal of the cardiac monitor.

Fig. 30 illustrates an embodiment of a connector suitable for when the signals between the catheter and the EP signal amplifiers have a different frequency than the PEF output.

Fig. 31 illustrates an embodiment of an interface connector.

Fig. 32 illustrates an embodiment of an interface connector having a component network.

Fig. 33 illustrates another embodiment of an interface connector having a component network.

Fig. 34 illustrates an embodiment of a tissue modification system for use with a patient.

Fig. 35 an embodiment of a tissue modification system for use with a patient wherein the treatment catheter comprises a particular conventional catheter.

Fig. 36 illustrates an embodiment of a treatment catheter configured to deliver "one-shot" therapy.

Figs. 37A-37B illustrate an embodiment of the delivery electrode configured to deliver "one shot" therapy, wherein the delivery electrode has a cup or funnel shape.

Figs. 38A-38B illustrate the application of the delivery electrode of 37A-37B to a surface.

Fig. 39 illustrates an embodiment of a delivery electrode as in Figs. 38A-38B wherein a portion of the plurality of wires is covered by insulation.

Fig. 40A provides a schematic illustration of a cross-section of a lumen of a pulmonary vein surrounded by cardiac tissue, and the treatment catheter is shown having a delivery electrode contacting the cardiac tissue in various locations via the lumen.

Fig. 40B is a graph illustrating the association between energy and treatment area depth when energy is delivered according to the methods illustrated in Fig. 40A.

Fig. 41 illustrates an embodiment of a delivery electrode comprising an initial single loop that forms a double layer rim.

Fig. 42 which provides a side view of the delivery electrode depicted in Fig. 41.

Figs. 43A-43E illustrate deployment of the delivery electrode of Figs. 41-42.

Fig. 44A illustrates an embodiment of a delivery electrode having two loops that extend at least partially around the rim so that the circular rim is comprised of two layers of wire in two portions.

Fig. 44B illustrates the two loops of Fig. 44A isolated for visualization.

Fig. 45 provides a side view of the delivery electrode depicted in Fig. 44.

Fig. 46A illustrates an embodiment of a delivery electrode having three loops that extend at least partially around the rim so that the circular rim is comprised of two layers of wire in three portions.

Fig. 46B illustrates the three loops of 16A isolated for visualization.

Fig. 47 provides a side view of the delivery electrode depicted in Fig. 46A.

## DETAILED DESCRIPTION

Devices, systems and methods are provided for treating conditions of the heart, particularly the occurrence of arrhythmias, more particularly atrial fibrillation, atrial flutter, ventricular tachycardia, Wolff-Parkinson-White syndrome, and/or atrioventricular nodal reentry tachycardia, to name a few. The devices, systems and methods deliver therapeutic energy to portions the heart to provide tissue modification, such as to the entrances to the pulmonary veins in the treatment of atrial fibrillation. Targeted specific anatomic locations include the superior vena cava, inferior vena cava, right pulmonary vein, left pulmonary vein, right atrium, right atrial appendage, left atrium, left atrial appendage, right ventricle, left ventricle, right ventricular outflow tract, left ventricular outflow tract, ventricular septum, left ventricular summit, regions of myocardial scar, myocardial infarction border zones, myocardial infarction channels, ventricular endocardium, ventricular epicardium, papillary muscles and the purkinje system, to name a few. Treatments are delivered at isolated sites or in a connected series of treatments. Types of treatment include the creation of left atrial roof line, left atrial posterior/inferior line, posterior wall isolation, lateral mitral isthmus line, septal mitral isthmus line, left atrial appendage, right sided cavotricuspid isthmus (CTI), pulmonary vein isolation, superior vena cava isolation, vein of Marshall, lesion creation using Complex Fractionated Atrial Electrograms (CFAE), lesion creation using Focal Impulse and Rotor Modulation (FIRM), and targeted ganglia ablation. Such tissue modification creates a conduction block within the tissue to prevent the transmission of aberrant electrical signals. The devices, systems and methods are typically used in an electrophysiology lab or controlled surgical suite equipped with fluoroscopy and advanced ECG recording and monitoring capability. An electrophysiologist (EP) is the intended primary user of the system. The electrophysiologist will be supported by a staff of trained nurses, technicians, and potentially other electrophysiologists. Generally, the tissue modification systems include a specialized catheter, a high voltage waveform generator and at least one distinct energy delivery algorithm. Additional accessories and equipment may be utilized. Example embodiments of specialized catheter designs are provided herein and include a variety of delivery types including focal delivery, "one-shot" delivery and various possible combinations. For illustration purposes a simplified design is provided when describing the overall system. Such a simplified design provides monopolar focal therapy. However, it may be appreciated that a variety of other embodiments are also provided.

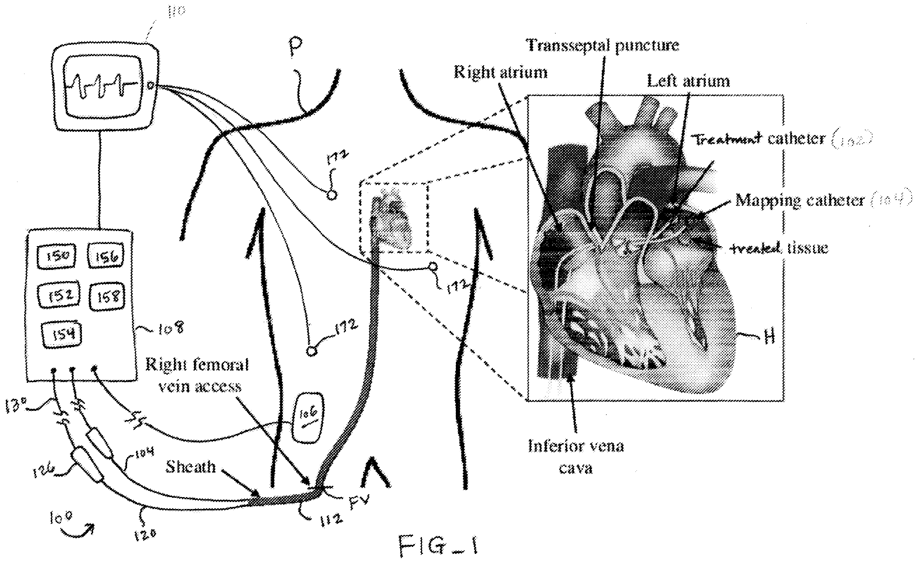

Fig. 1 illustrates an embodiment of a tissue modification system 100 comprising a treatment catheter 102, a mapping catheter 104, a return electrode 106, a waveform generator 108 and an external cardiac monitor 110. In this embodiment, the heart is accessed via the right femoral vein FV by a suitable access procedure, such as the Seldinger technique. Typically, a sheath 112 is inserted into the femoral vein FV which acts as a conduit through which various catheters and/or tools may be advanced, including the treatment catheter 102 and mapping catheter 104. It may be appreciated that in some embodiments, the treatment catheter 102 and mapping catheter 104 are combined into a single device. As illustrated in Fig. 1, the distal ends of the catheters 102, 104 are advanced through the inferior vena cava, through the right atrium, through a transseptal puncture and into the left atrium so as to access the entrances to the pulmonary veins. The mapping catheter 104 is used to perform cardiac mapping which refers to the process of identifying the temporal and spatial distributions of myocardial electrical potentials during a particular heart rhythm. Cardiac mapping during an aberrant heart rhythm aims at elucidation of the mechanisms of the heart rhythm, description of the propagation of activation from its initiation to its completion within a region of interest, and identification of the site of origin or a critical site of conduction to serve as a target for treatment. Once the desired treatment locations are identified, the treatment catheter 102 is utilized to deliver the treatment energy.

In this embodiment, the proximal end of the treatment catheter 102 is electrically connected with the waveform generator 108, wherein the generator 108 is software-controlled with regulated energy output that creates high frequency short duration energy delivered to the catheter 102. It may be appreciated that in various embodiments the output is controlled or modified to achieve a desired voltage, current, or combination thereof. In this embodiment, the proximal end of the mapping catheter 104 is also electrically connected with the waveform generator 108 and the electronics to perform the mapping procedure are included in the generator 108. However, it may be appreciated that the mapping catheter 104 may alternatively be connected with a separate external device having the capability of providing the mapping procedure, such as electroanatomic mapping (EAM) systems (e.g. CARTO® systems by Biosense Webster/Johnson & Johnson, EnSite™ systems by St. Jude Medical/Abbott, KODEX-EPD system by Philips, Rhythmia HDX™ system by Boston Scientific). Likewise, in some embodiments, a separate mapping catheter 104 is not used and the mapping features are built into the catheter 102.

In this embodiment, the generator 108 is connected with an external cardiac monitor 110 to allow coordinated delivery of energy with the cardiac signal sensed from the patient P. The generator synchronizes the energy output to the patient's cardiac rhythm. The cardiac monitor provides a trigger signal to the generator 108 when it detects the patient's cardiac cycle R-wave. This trigger signal, and the generator's algorithm, reliably synchronize the energy delivery with the patient's cardiac cycle to decrease the potential for arrhythmia due to energy delivery. Typically, a footswitch allows the user to initiate and control the delivery of the energy output. The generator user interface (UI) provides both audio and visual information to the user regarding energy delivery and the generator operating status.

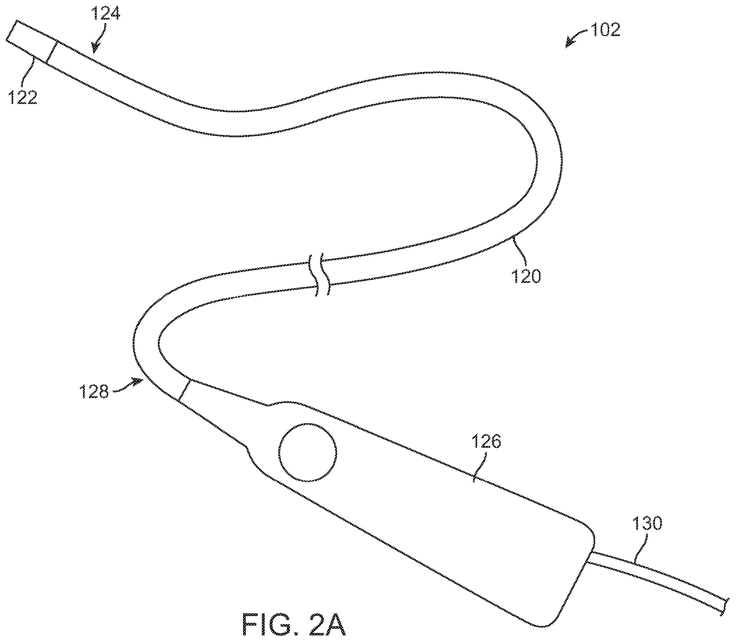

In this embodiment, the treatment catheter 102 is designed to be monopolar, wherein the distal end of the catheter 108 has as a delivery electrode 122 and the return electrode 106 is positioned upon the skin outside the body, typically on the thigh, lower back or back. Fig. 2A illustrates an embodiment of a treatment catheter 102 configured to deliver focal therapy. In this embodiment, the catheter 102 comprises an elongate shaft 120 having a delivery electrode 122 near its distal end 124 and a handle 126 near its proximal end 128. The delivery electrode 122 is shown as a "solid tip" electrode having a cylindrical shape with a distal face having a continuous surface. In some embodiments, the cylindrical shape has a diameter across its distal face of approximately 2-3mm and a length along the shaft 120 of approximately 1mm, 2mm, 1-2mm, 3mm, 4mm, 3-4mm, 5mm, 6mm, 7mm, 8mm, 9mm, 10mm, etc. It may be appreciated that such electrodes are typically hollow yet are referred to as solid due to visual appearance. In some embodiments, the catheter 102 has an overall length of 50-150cm, preferably 100-125cm, more preferably 110-115cm. Likewise, in some embodiments, it has a 7 Fr outer diameter 3-15Fr, preferably 4-12Fr, more preferably 7-8.5Fr. It may be appreciated that in some embodiments, the shaft 120 has a deflectable end portion 121 and optionally the deflectable end portion 121 may have a length of 50-105mm resulting in curves with diameters ranging from approximately 15 to 55mm. Deflection may be achieved by a variety of mechanism including a pull-wire which extends to the handle 126. Thus, the handle 126 is used to manipulate the catheter 102, particularly to steer the distal end 124 during delivery and treatment. Energy is provided to the catheter 102, and therefore to the delivery electrode 122, via a cable 130 that is connectable to the generator 108.

Pulsed electric fields (PEFs) are provided by the generator 108 and delivered to the tissue through the delivery electrode 122 placed on or near the targeted tissue area. It may be appreciated that in some embodiments, the delivery electrode 122 is positioned in contact with a conductive substance which is likewise in contact with the targeted tissue. Such solutions may include isotonic or hypertonic solutions. These solutions may further include adjuvant materials, such as chemotherapy or calcium, to further enhance the treatment effectiveness both for the focal treatment as well as potential regional infiltration regions of the targeted tissue types. High voltage, short duration biphasic electric pulses are then delivered through the electrode 122 in the vicinity of the target tissue. These electric pulses are provided by at least one energy delivery algorithm 152. In some embodiments, each energy delivery algorithm 152 prescribes a signal having a waveform comprising a series of energy packets wherein each energy packet comprises a series of high voltage pulses. In such embodiments, the algorithm 152 specifies parameters of the signal such as energy amplitude (e.g., voltage) and duration of applied energy, which is comprised of the number of packets, number of pulses within a packet, and the fundamental frequency of the pulse sequence, to name a few. Additional parameters may include switch time between polarities in biphasic pulses, dead time between biphasic cycles, and rest time between packets, which will be described in more detail in later sections. There may be a fixed rest period between packets, or packets may be gated to the cardiac cycle and are thus variable with the patient's heart rate. There may be a deliberate, varying rest period algorithm or no rest period may also be applied between packets. A feedback loop based on sensor information and an auto-shutoff specification, and/or the like, may be included.

It may be appreciated that in various embodiments the treatment catheter 102 includes a variety of specialized features. For example, in some embodiments, the catheter 102 includes a mechanism for real-time measurement of the contact force applied by the catheter tip to a patient's heart wall during a procedure. In some embodiments, this mechanism is included in the shaft 120 and comprises a tri-axial optical force sensor which utilizes white light interferometry. By monitoring and modifying the applied force throughout the procedure, the user is able to better control the catheter 102 so as to create more consistent and effective lesions.

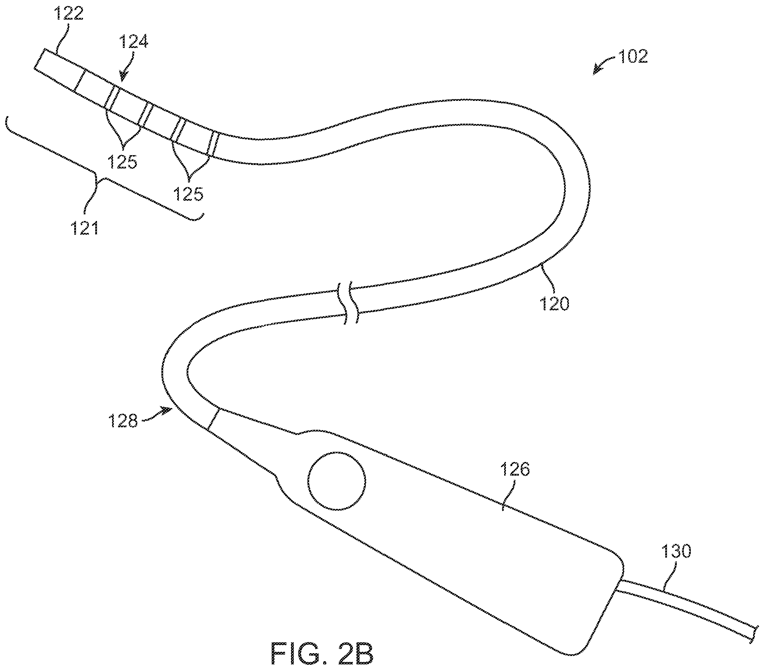

In some embodiments, the catheter 102 includes one or more additional electrodes 125 (e.g. ring electrodes) positioned along the shaft 120, such as illustrated in Fig. 2B, proximal to the delivery electrode 122. In some embodiments, some or all of the additional electrodes can be used for stimulating and recording (for electrophysiological mapping), so a separate cardiac mapping catheter is not needed when using catheter 102 for lesion creation, or for other purposes such as sensing, etc.

In some embodiments, the catheter 102 includes a thermocouple temperature sensor, optionally embedded in the delivery electrode 122. Likewise, in some embodiments the catheter 102 includes a lumen which may be used for irrigation and/or suction. Typically, the lumen connects with one or more ports along the distal end of the catheter 102, such as for the injection of isotonic saline solution to irrigate or for the removal of, for example, microbubbles.

In some embodiments, the catheter 102 includes one or more sensors that can be used to determine temperature, impedance, resistance, capacitance, conductivity, permittivity, and/or conductance, to name a few. In some embodiments, one or more of the electrodes act as the one or more sensors. In other embodiments, the one or more sensors are separate from the electrodes. Sensor data can be used to plan the therapy, monitor the therapy and/or provide direct feedback via the processor 154, which can then alter the energy-delivery algorithm 152. For example, impedance measurements can be used to determine not only the initial dose to be applied but can also be used to determine the need for further treatment, or not.

Referring back to Fig. 1, in this embodiment the generator 108 includes a user interface 150, one or more energy delivery algorithms 152, a processor 154, a data storage/retrieval unit 156 (such as a memory and/or database), and an energy-storage sub-system 158 which generates and stores the energy to be delivered. In some embodiments, one or more capacitors are used for energy storage/delivery, however any other suitable energy storage element may be used. In addition, one or more communication ports are included.

In some embodiments, the generator 108 includes three sub-systems: 1) a high-energy storage system, 2) a high-voltage, medium-frequency switching amplifier, and 3) the system controller, firmware, and user interface. In this embodiment, the system controller includes a cardiac synchronization trigger monitor that allows for synchronizing the pulsed energy output to the patient's cardiac rhythm. The generator takes in alternating current (AC) mains to power multiple direct current (DC) power supplies. The generator's controller can cause the DC power supplies to charge a high-energy capacitor storage bank before energy delivery is initiated. At the initiation of therapeutic energy delivery, the generator's controller, high-energy storage banks and a bi-phasic pulse amplifier can operate simultaneously to create a high-voltage, medium frequency output.

It will be appreciated that a multitude of generator electrical architectures may be employed to execute the energy delivery algorithms. In particular, in some embodiments, advanced switching systems are used which are capable of directing the pulsed electric field circuit to the energy delivering electrodes separately from the same energy storage and high voltage delivery system. Further, generators employed in advanced energy delivery algorithms employing rapidly varying pulse parameters (e.g., voltage, frequency, etc.) or multiple energy delivery electrodes may utilize modular energy storage and/or high voltage systems, facilitating highly customizable waveform and geographical pulse delivery paradigms. It should further be appreciated that the electrical architecture described herein above is for example only, and systems delivering pulsed electric fields may or may not include additional switching amplifier components.

The user interface 150 can include a touch screen and/or more traditional buttons to allow for the operator to enter patient data, select a treatment algorithm (e.g., energy delivery algorithm 152), initiate energy delivery, view records stored on the storage/retrieval unit 156, and/or otherwise communicate with the generator 108.

In some embodiments, the user interface 150 is configured to receive operator-defined inputs. The operator-defined inputs can include a duration of energy delivery, one or more other timing aspects of the energy delivery pulse, power, and/or mode of operation, or a combination thereof. Example modes of operation can include (but are not limited to): system initiation and self-test, operator input, algorithm selection, pre-treatment system status and feedback, energy delivery, post energy delivery display or feedback, treatment data review and/or download, software update, or any combination or subcombination thereof.

As mentioned, in some embodiments the system 100 also includes a mechanism for acquiring an electrocardiogram (ECG), such as an external cardiac monitor 110, in situations wherein cardiac synchronization is desired. Example cardiac monitors are available from AccuSync Medical Research Corporation and Ivy Biomedical Systems, Inc. In some embodiments, the external cardiac monitor 110 is operatively connected to the generator 108. The cardiac monitor 110 can be used to continuously acquire an ECG signal. External electrodes 172 may be applied to the patient P to acquire the ECG. The generator 108 analyzes one or more cardiac cycles and identifies the beginning of a time period during which it is safe to apply energy to the patient P, thus providing the ability to synchronize energy delivery with the cardiac cycle. In some embodiments, this time period is within milliseconds of the R wave (of the ECG QRS complex) to avoid induction of an arrhythmia, which could occur if the energy pulse is delivered on a T wave. It will be appreciated that such cardiac synchronization is typically utilized when using monopolar energy delivery, however it may be utilized as part of other energy delivery methods.

In some embodiments, the processor 154, among other activities, modifies and/or switches between the energy-delivery algorithms, monitors the energy delivery and any sensor data, and reacts to monitored data via a feedback loop. In some embodiments, the processor 154 is configured to execute one or more algorithms for running a feedback control loop based on one or more measured system parameters (e.g., current), one or more measured tissue parameters (e.g., impedance), and/or a combination thereof.

The data storage/retrieval unit 156 stores data, such as related to the treatments delivered, and can optionally be downloaded by connecting a device (e.g., a laptop or thumb drive) to a communication port. In some embodiments, the device has local software used to direct the download of information, such as, for example, instructions stored on the data storage/retrieval unit 156 and executable by the processor 154. In some embodiments, the user interface 150 allows for the operator to select to download data to a device and/or system such as, but not limited to, a computer device, a tablet, a mobile device, a server, a workstation, a cloud computing apparatus/system, and/or the like. The communication ports, which can permit wired and/or wireless connectivity, can allow for data download, as just described but also for data upload such as uploading a custom algorithm or providing a software update.

As described herein, a variety of energy delivery algorithms 152 are programmable, or can be pre-programmed, into the generator 108, such as stored in memory or data storage/retrieval unit 156. Alternatively, energy delivery algorithms can be added into the data storage/retrieval unit to be executed by processor 154. Each of these algorithms 152 may be executed by the processor 154.

It may be appreciated that in some embodiments the system 100 includes an automated treatment delivery algorithm that dynamically responds and adjusts and/or terminates treatment in response to inputs such as temperature, impedance at various voltages or AC frequencies, treatment duration or other timing aspects of the energy delivery pulse, treatment power and/or system status.

As mentioned, in some embodiments, the cardiac monitor provides a trigger signal to the generator 108 when it detects the patient's cardiac cycle R-wave. This trigger signal, and the generator's algorithm, reliably synchronize the energy delivery with the patient's cardiac cycle to decrease the potential for arrhythmia due to energy delivery. This trigger is within milliseconds of the peak of the R wave (of the ECG QRS complex) to avoid induction of an arrhythmia, which could occur if the energy pulse is delivered on a T wave, and also to ensure that energy delivery occurs at a consistent phase of cardiac contraction. It will be appreciated that such cardiac synchronization is typically utilized when using monopolar energy delivery, however it may be utilized as part of other energy delivery methods.

In this embodiment, the generator 108 is connected with an external cardiac monitor 110 to allow coordinated delivery of energy with the cardiac signal sensed from the patient P.

In some embodiments, the generator 180 receives feedback from the cardiac monitor 110 and responds based on the received information. In some embodiments, the generator 180 receives information regarding the heart rate of the patient and either halts delivery of energy or modifies the energy delivery, such as by selecting a different energy delivery algorithm 152. In some embodiments, the generator 180 halts delivery of energy when the heart rate reaches or drops below a threshold value, such as 30 beats per minute (bpm) or 20 bpm. Optionally, the generator may provide an indicator, such as a visual or auditory indicator, when the heart rate reaches or drops below a lower threshold value, such as providing a flashing yellow light when the heart rate reaches 30 bpm and a solid red light when the heart rate reaches 20 bpm. Such safety measures ensure that the treatment energy is not delivered at an inappropriate time given that low sporadic heart rates may indicate erroneous readings.

In some embodiments, the generator 108 modifies the energy delivery based on the information from the cardiac monitor 110. For example, in some embodiments, energy delivery is provided in a 1:1 ratio when the heart rate is in a predetermined range, such as between 40 bpm and 120 bpm. This involves delivery of PEF energy at the appropriate interval of each heart beat. In some embodiments, the generator 108 modifies the energy delivery if the heart rate exceeds this range, such as if the heart rate exceeds 120 bpm. In some embodiments, the energy delivery is modified to a 2:1 ratio (two heartbeats: one delivery) wherein PEF energy is delivered at the appropriate interval of every other heart beat. It may be appreciated that various ratios of the form m:n (where m and n are integers) may be utilized, such as 3:1, 3:2, 4:1, 4:3 5:1, etc. It may also be appreciated that in some embodiments the heart rate may be paced to achieve a desired heart rate. Such pacing may be provided by a separate or integrated pacemaker. In some embodiments, such pacing is provided by a catheter positioned in the coronary sinus that is used for recording during procedures but is also available for pacing. Such pacing may be triggered by the generator 108 or the cardiac monitor 110.

In some embodiments, the generator 108 halts energy delivery or modifies the energy delivery based on information from other sources, such as from various sensors, including temperature sensors, impedance sensors, contact or contact force sensors, etc. In some embodiments, the generator 108 modifies energy delivery based on sensed temperature (e.g. on the catheter 102, in nearby tissue, in nearby structures, etc.). In some embodiments, energy delivery is modified to a 2:1 ratio, wherein PEF energy is delivered at the appropriate interval of every other heart beat, when the temperature reaches a predetermined threshold value. Such a modification reduces any small thermal effects, thereby reducing sensed temperature. It may be appreciated that various ratios may be utilized, such as 3:1, 3:2, 4:3, 4:1, 5:1, etc.

As mentioned previously, one or more energy delivery algorithms 152 are programmable, or can be pre-programmed, into the generator 108 for delivery to the patient P. The one or more energy delivery algorithms 152 specify electric signals which provide energy delivered to the cardiac tissue which are non-thermal (e.g. below a threshold for thermal ablation; below a threshold for inducing coagulative thermal damage), reducing or avoiding inflammation, and/or preventing denaturation of stromal proteins in the luminal structures. It may be appreciated that the non-thermal energy is also not cryogenic (i.e. it is above a threshold for thermal damage caused by freezing). Thus, the temperature of the target tissue remains in a range between a baseline body temperature (such as 35°C-37°C but can be as low as 30°C) and a threshold for thermal ablation. Thus, targeted ranges of tissue temperature include 30-65°C, 30-60°C, 30-55°C, 30-50°C, 30-45°C, 30-35°C. Thus, lesions in the heart tissue are not created by thermal injury as the temperature of the tissue remains below a threshold for thermal ablation (e.g. 65°C). In addition, the impedance of the tissue typically remains below a threshold generated by thermal ablation. Charring and thermal injury of tissue changes the conductivity of the heart tissue. This increase in impedance/reduction in conductivity often indicates thermal injury and reduces the ability of the tissue to receive further energy. In some instances, the impedance of the system circuit from the cathode to the anode remains in the range of 25-250Ω, or 50-200 Ω during delivery of PEF energy. In general, the algorithms 152 are tailored to affect tissue to a pre-determined depth and/or volume and/or to target specific types of cellular responses to the energy delivered. However, it may be appreciated that the pulsed electric field energy described herein may be utilized more liberally than other types of energy, such as those that cause thermal injury, without negative effects. For instance, since the energy does not cause thermal injury, tissue can be over-treated to ensure sufficient lesion formation. For example, in a tissue layer that is 2mm thick, energy sufficient to create a lesion having a depth of 6mm can be applied to the tissue to ensure a transmural lesion. Typically, the additional energy is dissipated away from nearby critical structures through transverse tissue planes. In particular, the pericardial fluid surrounding the heart serves to dissipate energy, protecting extracardiac structures, such as the esophagus, phrenic nerve, coronary arteries, lungs, and bronchioles, from injury. This is not the case when delivering energy that creates lesions by thermal injury. In those cases, the propagation of conductive thermal energy beyond the targeted myocardial tissue can result in thermal injury to non-targeted extracardiac structures. Excessive thermal injury to the esophagus may result in esophageal ulcers that can degrade to a life-threatening atrio-esophageal fistula. Thermal injury to the phrenic nerve may result in permanent diaphragmatic paralysis leading to permanent shortness of breath and fatigue. Thermal injury to the coronary arteries can result in coronary spasm that can lead to temporary, or even permanent, chest pressure/pain. In addition, thermal lesions in the heart, in the region of the pulmonary veins can lead to pulmonary vein stenosis. Pulmonary vein stenosis is a known complication of radiofrequency ablation near the pulmonary veins in patients with atrial fibrillation. This pathologic process is related to thermal injury to the tissue that induces post-procedure fibrosis and scaring. Stenosis has been described in patients treated with many forms of thermal energy, including radiofrequency energy and cryoablation.

Since the PEF lesions described herein are not created by thermal injury, rates of "false positive" confirmation of electrical conduction blocks are also reduced. Thermal injury may result in acute myocardial edema (i.e. tissue fluid accumulation and swelling). When testing electrical conductivity across an area of thermally ablated tissue, the tissue may appear to block electrical conduction however such blocking may simply be the result of temporary edema. After a period of recovery to allow the swelling to subside, this area of treated tissue will no longer have transmural, non-conduction. In addition, acute edema due to thermal injury also diminishes the ability to re-treat an area of tissue. Once an area of tissue has undergone an amount of thermal injury, the resulting edema changes the resistive and conductive thermal properties of the tissue. Therefore, effects similar to the initial response in the tissue are difficult to obtain. Thus, any attempted re-treatment is less effective both acutely and chronically. These issues are avoided with the delivery of the energy described herein.

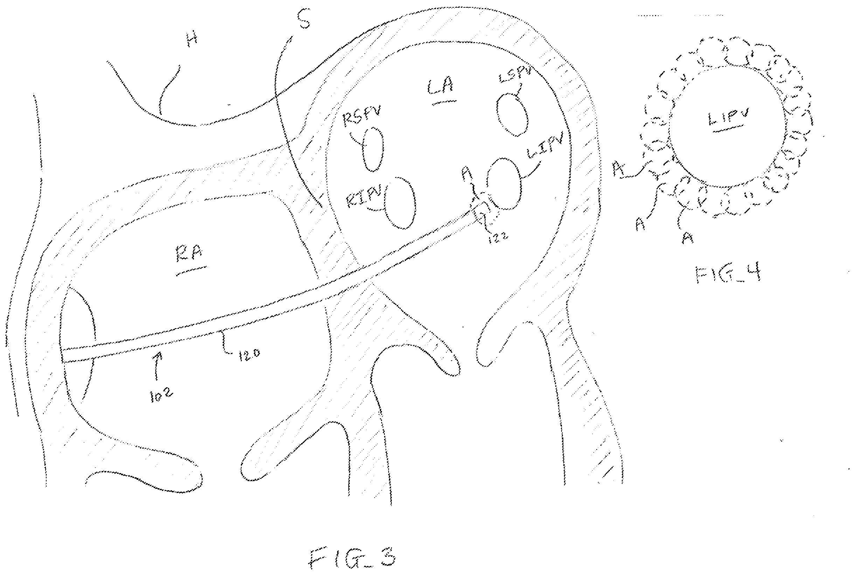

Fig. 3 illustrates a portion of the heart H showing a cut-away of the right atrium RA and left atrium LA in the treatment of atrial fibrillation. The largest pulmonary veins are the four main pulmonary veins (right superior pulmonary vein RSPV, right inferior pulmonary vein RIPV, left superior pulmonary vein LSPV and left inferior pulmonary vein LIPV), two from each lung that drain into the left atrium LA of the heart H. Each pulmonary vein is linked to a network of capillaries in the alveoli of each lung and bring oxygenated blood to the left atrium LA. The left atrial musculature extends from the left atrium LA and envelopes the proximal pulmonary veins. The superior veins, which have longer muscular sleeves, have been reported to be more arrhythmogenic than the inferior veins. In general, the length of the pulmonary vein sleeves varies between 13 mm and 25 mm. Pulmonary vein morphology has been reported to influence arrhythmogenesis. Likewise, cellular electrophysiology and other aspects of the pulmonary veins are associated with arrhythmogenesis and propagation.

A variety of methods are used to determine which tissue is targeted for treatment, such as anatomical indications and cardiac mapping. Typically, a mapping catheter is chosen to desirably fit the pulmonary vein, adapting to the size and anatomical form of the pulmonary vein. The mapping catheter allows recording of the electrograms from the ostium of the pulmonary vein and from deep within the pulmonary vein; these electrograms are displayed and timed for the user. The treatment catheter 102 is initially placed deep within the pulmonary vein and gradually withdrawn to the ostium, proximal to the mapping catheter. Mapping and treatment then commences.

The current understanding of pulmonary vein electrophysiology is that most of the fibers in the pulmonary vein are circular and do not carry conduction into the vein. The electrical conduction pathways are longitudinal fibers which extend between the left atrium LA and the pulmonary vein. Pulmonary vein isolation is achieved by ablation of these connecting longitudinal fibers. For the left-sided pulmonary veins, pacing of the distal coronary sinus tends to increase the separation of the atrial signal and the pulmonary vein potential making these more electrically visible. The signals from within the pulmonary vein are evaluated. Each individual signal consists of a far field atrial signal, which is generally of low amplitude, and a sharp local pulmonary vein spike. The earliest pulmonary vein spike represents the site of the connection of the pulmonary vein and atrium. If the pulmonary vein spike and the atrial potential are examined, on some of the poles of the mapping catheter, these electrograms are widely separated, at other sites there will be a fusion potential of the atrial and PV signal. The latter indicate the sites of the longitudinal fibers and the potential sites for treatment.

In some embodiments, the tissue surrounding the opening of the left inferior pulmonary vein LIPV is treated in a point by point fashion with the use of the treatment catheter 102 (with assistance of mapping) to create a circular treatment zone around the left inferior pulmonary vein LIPV, as illustrated in Fig. 3. In some instances, specialized navigation software can be used to allow appropriate positioning of the treatment catheter 120. The delivery electrode 122 is positioned near or against the target tissue area, and energy is provided to the delivery electrode 122 so as to create a treatment area A. Since the energy is delivered to a localized area (focal delivery), the electrical energy is concentrated over a smaller surface area, resulting in stronger effects than delivery through an electrode extending circumferentially around the lumen or ostium. It also forces the electrical energy to be delivered in a staged regional approach, mitigating the potential effect of preferential current pathways through the surrounding tissue. These preferential current pathways are regions with electrical characteristics that induce locally increased electric current flow therethrough rather than through adjacent regions. Such pathways can result in an irregular electric current distribution around the circumference of a targeted lumen, which thus can distort the electric field and cause an irregular increase in treatment effect for some regions and a lower treatment effect in other regions. This may be mitigated or avoided with the use of focal therapy which stabilizes the treatment effect around the circumference of the targeted region. Thus, by providing the energy to certain regions at a time, the electrical energy is "forced" across different regions of the circumference, ensuring an improved degree of treatment circumferential regularity. Fig. 4 illustrates the repeated application of energy in point by point fashion around the left inferior pulmonary vein LIPV with the use of the treatment catheter 102 to create a circular treatment zone. As illustrated, in this embodiment each treatment area A overlaps an adjacent treatment area A so as to create a continuous treatment zone. The size and depth of each treatment area A may depend on a variety of factors, such as parameter values, treatment times, tissue characteristics, etc. It may be appreciated that the number of treatment areas A may vary depending on a variety of factors, particularly the unique conditions of each patient's anatomy and electrophysiology. In some embodiments, the number of treatment areas A include one, two, three, four, five, six, seven, eight, nine, ten, fifteen, twenty, twenty five, thirty or more.

When all the electrical connections between the atrium and the vein have been treated, there is electrical silence within the pulmonary vein, with only the far field atrial signal being recorded. Occasionally spikes of electrical activity are seen within the pulmonary vein with no conduction to the rest of the atrium; these clearly demonstrate electrical discontinuity of the vein from the rest of the atrial myocardium.

Additional treatment areas can be created at other locations to treat arrhythmias in either the right or left atrium dependent on the clinical presentation. Testing is then performed to ensure that each targeted pulmonary vein is effectively isolated from the body of the left atrium.

Energy Delivery Algorithms

It may be appreciated that a variety of energy delivery algorithms 152 may be used. In some embodiments, the algorithm 152 prescribes a signal having a waveform comprising a series of energy packets wherein each energy packet comprises a series of high voltage pulses. In such embodiments, the algorithm 152 specifies parameters of the signal such as energy amplitude (e.g., voltage) and duration of applied energy, which is comprised of the number of packets, number of pulses within a packet, and the fundamental frequency of the pulse sequence, to name a few. Additional parameters may include switch time between polarities in biphasic pulses, dead time between biphasic cycles, and rest time between packets, which will be described in more detail in later sections. There may be a fixed rest period between packets, or packets may be gated to the cardiac cycle and are thus variable with the patient's heart rate. There may be a deliberate, varying rest period algorithm or no rest period may also be applied between packets. A feedback loop based on sensor information and an auto-shutoff specification, and/or the like, may be included.

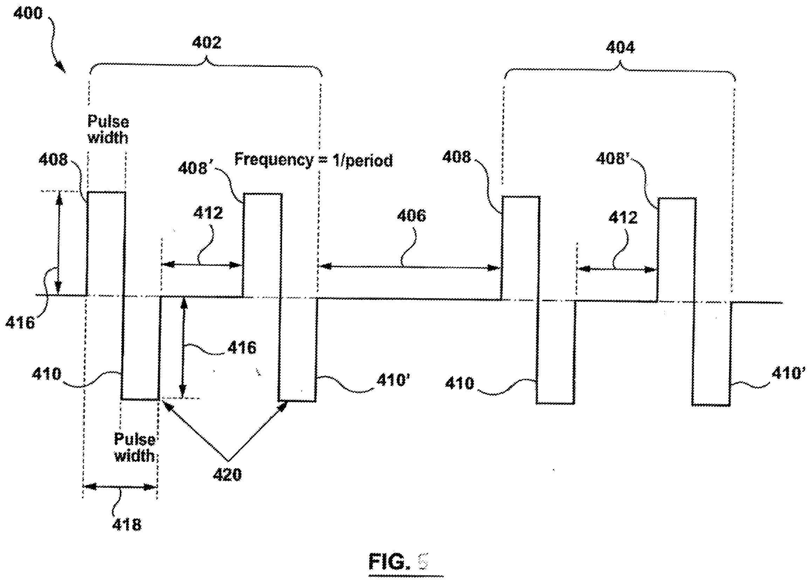

Fig. 5 illustrates an embodiment of a waveform 400 of a signal prescribed by an energy delivery algorithm 152. Here, two packets are shown, a first packet 402 and a second packet 404, wherein the packets 402, 404 are separated by a rest period 406. In this embodiment, each packet 402, 404 is comprised of a first biphasic cycle (comprising a first positive pulse peak 408 and a first negative pulse peak 410) and a second biphasic cycle (comprising a second positive pulse peak 408' and a second negative pulse peak 410'). The first and second biphasic pulses are separated by dead time 412 (i.e. a pause) between each biphasic cycle. In this embodiment, the biphasic pulses are symmetric so that the set voltage 416 is the same for the positive and negative peaks. Here, the biphasic, symmetric waves are also square waves such that the magnitude and time of the positive voltage wave is approximately equal to the magnitude and time of the negative voltage wave.

### A. Voltage

The voltages used and considered may be the tops of square-waveforms, may be the peaks in sinusoidal or sawtooth waveforms, or may be the RMS voltage of sinusoidal or sawtooth waveforms. In some embodiments, the energy is delivered in a monopolar fashion and each high voltage pulse or the set voltage 416 is between about 500V to 10,000V, particularly about 1000V-2000V, 2000V-3000V, 3000V-3500V, 3500V-4000V, 3500V-5000V, 3500V-6000V, including all values and subranges in between including about 1000V, 2000V, 2500V, 2800V, 3000V, 3300V, 3500V, 3700V, 4000V, 4500V, 5000V, 5500V, 6000V to name a few.

It may be appreciated that the set voltage 416 may vary depending on whether the energy is delivered in a monopolar or bipolar fashion. In bipolar delivery, a lower voltage may be used due to the smaller, more directed electric field. The bipolar voltage selected for use in therapy is dependent on the separation distance of the electrodes, whereas the monopolar electrode configurations that use one or more distant dispersive pad electrodes may be delivered with less consideration for exact placement of the catheter electrode and dispersive electrode placed on the body. In monopolar electrode embodiments, larger voltages are typically used due to the dispersive behavior of the delivered energy through the body to reach the dispersive electrode, on the order of 10cm to 100cm effective separation distance. Conversely, in bipolar electrode configurations, the relatively close active regions of the electrodes, on the order of 0.5mm to 10cm, including 1mm to 1cm, results in a greater influence on electrical energy concentration and effective dose delivered to the tissue from the separation distance. For instance, if the targeted voltage-to-distance ratio is 3000 V/cm to evoke the desired clinical effect at the appropriate tissue depth (1.3mm), if the separation distance is changed from 1mm to 1.2mm, this would result in a necessary increase in treatment voltage from 300 to about 360 V, a change of 20%.

### B. Frequency

It may be appreciated that the number of biphasic cycles per second of time is the frequency when a signal is continuous. In some embodiments, biphasic pulses are utilized to reduce undesired muscle stimulation, particularly cardiac muscle stimulation. In other embodiments, the pulse waveform is monophasic and there is no clear inherent frequency. Instead, a fundamental frequency may be considered by doubling the monophasic pulse length to derive the frequency. In some embodiments, the signal has a frequency in the range 50kHz-1MHz, more particularly 50kHz - 1000kHz. It may be appreciated that at some voltages, frequencies at or below 100-250 kHz may cause undesired muscle stimulation. Therefore, in some embodiments, the signal has a frequency in the range of 300-800kHz, 400-800 kHz or 500-800 kHz, such as 300kHz, 400kHz, 450kHz, 500 kHz, 550 kHz, 600 kHz, 650 kHz, 700 kHz, 750 kHz, 800 kHz. In addition, cardiac synchronization is typically utilized to reduce or avoid undesired cardiac muscle stimulation during sensitive rhythm periods. It may be appreciated that even higher frequencies may be used with components which minimize signal artifacts.

### C. Voltage-Frequency Balancing

The frequency of the waveform delivered may vary relative to the treatment voltage in synchrony to retain adequate treatment effect. Such synergistic changes would include the decrease in frequency, which evokes a stronger effect, combined with a decrease in voltage, which evokes a weaker effect. For instance, in some cases the treatment may be delivered using 3000 V in a monopolar fashion with a waveform frequency of 600kHz, while in other cases the treatment may be delivered using 2000 V with a waveform frequency of 400 kHz.

### D. Packets

As mentioned, the algorithm 152 typically prescribes a signal having a waveform comprising a series of energy packets wherein each energy packet comprises a series of high voltage pulses. The cycle count 420 is half the number of pulses within each biphasic packet. Referring to Fig. 5, the first packet 402 has a cycle count 420 of two (i.e. four biphasic pulses). In some embodiments, the cycle count 420 is set between 2 and 1000 per packet, including all values and subranges in between. In some embodiments, the cycle count 420 is 5-1000 per packet, 2-10 per packet, 2-20 per packet, 2-25 per packet, 10-20 per packet, 20 per packet, 20-30 per packet, 25 per packet, 20-40 per packet, 30 per packet, 20-50 per packet, 30-60 per packet, up to 60 per packet, up to 80 per packet, up to 100 per packet, up to 1,000 per packet or up to 2,000 per packet, including all values and subranges in between.

The packet duration is determined by the cycle count, among other factors. For a matching pulse duration (or sequence of positive and negative pulse durations for biphasic waveforms), the higher the cycle count, the longer the packet duration and the larger the quantity of energy delivered. In some embodiments, packet durations are in the range of approximately 50 to 1000 microseconds, such as 50 $\mu$s, 60 $\mu$s, 70 $\mu$s, 80 $\mu$s, 90 $\mu$s, 100 $\mu$s, 125 $\mu$s, 150 $\mu$s, 175 $\mu$s, 200 $\mu$s, 250 $\mu$s, 100 to 250 $\mu$s, 150 to 250 $\mu$s, 200 to 250 $\mu$s, 500 to 1000 $\mu$s to name a few . In other embodiments, the packet durations are in the range of approximately 100 to 1000 microseconds, such as 150 μs, 200 μs, 250 μs, 500 μs, or 1000 μs.

The number of packets delivered during treatment, or packet count, typically includes 1 to 250 packets including all values and subranges in between. In some embodiments, the number of packets delivered during treatment comprises 10 packets, 15 packets, 20 packets, 25 packets, 30 packets or greater than 30 packets.

E. Rest Period

In some embodiments, the time between packets, referred to as the rest period 406, is set between about 0.001 seconds and about 5 seconds, including all values and subranges in between. In other embodiments, the rest period 406 ranges from about 0.01-0.1 seconds, including all values and subranges in between. In some embodiments, the rest period 406 is approximately 0.5ms - 500ms, 1-250ms, or 10-100ms to name a few.

F. Batches

In some embodiments, the signal is synced with the cardiac rhythm so that each packet is delivered synchronously within a designated period relative to the heartbeats, thus the rest periods coincide with the heartbeats. It may be appreciated that the packets that are delivered within each designated period relative to the heartbeats may be considered a batch or bundle. Thus, each batch has a desired number of packets so that at the end of a treatment period, the total desired number of packets have been delivered. Each batch may have the same number of packets, however in some embodiments, batches have varying numbers of packets.

In some embodiments, only one packet is delivered between heartbeats. In such instances, the rest period may be considered the same as the period between batches. However, when more than one packet is delivered between batches, the rest time is typically different than the period between batches. In such instances, the rest time is typically much smaller than the period between batches. In some embodiments, each batch includes 1-10 packets, 1-5 packets, 1-4 packets, 1-3 packets, 2-3 packets, 2 packets, 3 packets, 4 packets 5 packets, 5-10 packets, to name a few. In some embodiments, each batch has a period of 0.5ms-1sec, 1ms-1sec, 10ms-1sec, 10ms-100ms, to name a few. In some embodiments, the period between batches is variable, depending on the heart rate of the patient. In some instances, the period between batches is 0.25-5 seconds.

Treatment of a tissue area ensues until a desired number of batches are delivered to the tissue area. In some embodiments, 2-50 batches are delivered per treatment, wherein a treatment is considered treatment of a particular tissue area. In other embodiments, treatments include 5-40 batches, 5-30 batches, 5-20 batches, 5-10 batches, 5 batches, 6 batches, 7 batches, 8 batches, 9 batches, 10 batches, 10-15 batches, etc.

G. Switch Time and Dead Time

A switch time is a delay or period of no energy that is delivered between the positive and negative peaks of a biphasic pulse, as illustrated in Fig. 5. In some embodiments, the switch time ranges between about 0 to about 1 microsecond, including all values and subranges in between. In other embodiments, the switch time ranges between 1 and 20 microseconds, including all values and subranges in between. In other embodiments, the switch time ranges between about 2 to about 8 microsecond, including all values and subranges in between.

Delays may also be interjected between each biphasic cycle, referred as "dead-time". Dead time occurs within a packet, but between biphasic pulses. This is in contrast to rest periods which occur between packets. In other embodiments, the dead time 412 is in a range of approximately 0 to 0.5 microseconds, 0 to 10 microseconds, 2 to 5 microseconds, 0 to 20 microseconds, about 0 to about 100 microseconds, or about 0 to about 100 milliseconds, including all values and subranges in between. In some embodiments, the dead time 412 is in the range of 0.2 to 0.3 microseconds. Dead time may also be used to define a period between separate, monophasic, pulses within a packet.

Delays, such as switch times and dead times, are introduced to a packet to reduce the effects of biphasic cancellation within the waveform. In some instances, the switch time and dead time are both increased together to strengthen the effect. In other instances, only switch time or only dead time are increased to induce this effect.

G. Waveforms

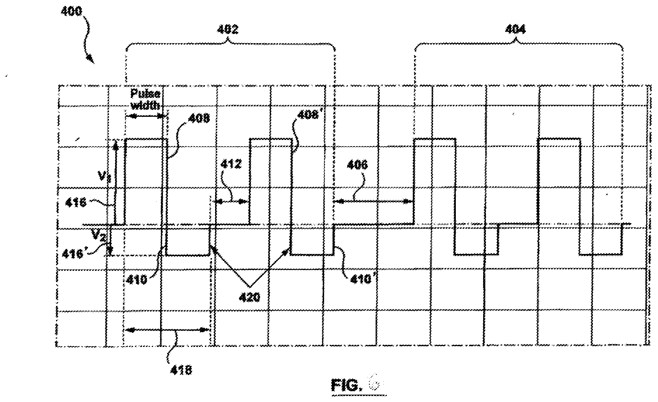

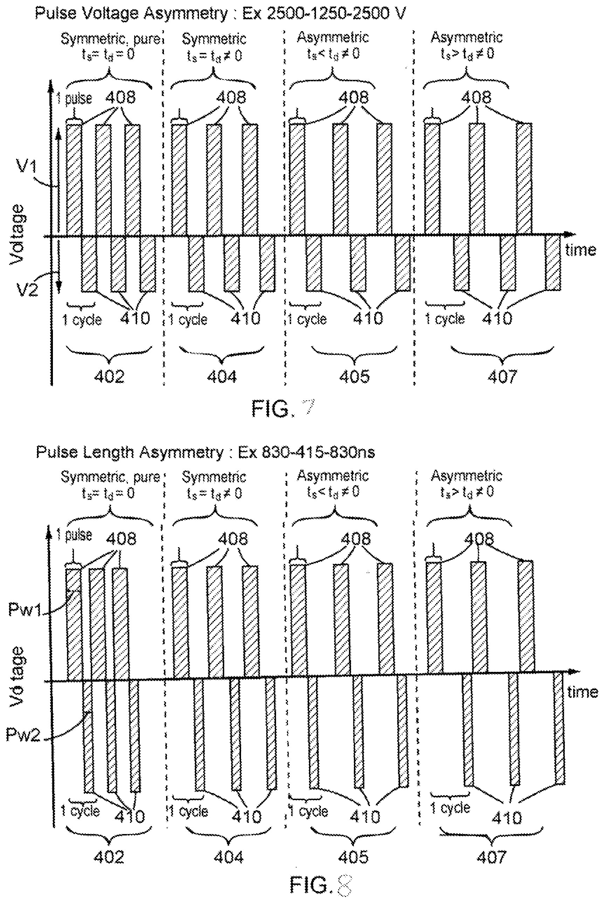

Fig. 5 illustrated an embodiment of a waveform 400 having symmetric pulses such that the voltage and duration of pulse in one direction (i.e., positive or negative) is equal to the voltage and duration of pulse in the other direction. Fig. 6 illustrates an example waveform 400 prescribed by another energy delivery algorithm 152 wherein the waveform 400 has voltage imbalance. Here, two packets are shown, a first packet 402 and a second packet 404, wherein the packets 402, 404 are separated by a rest period 406. In this embodiment, each packet 402, 404 is comprised of a first biphasic cycle (comprising a first positive pulse peak 408 having a first voltage V1 and a first negative pulse peak 410 having a second voltage V2) and a second biphasic cycle (comprising a second positive pulse peak 408' having first voltage V1 and a second negative pulse peak 410' having a second voltage V2). Here the first voltage V1 is greater than the second voltage V2. The first and second biphasic cycles are separated by dead time 412 between each pulse. Thus, the voltage in one direction (i.e., positive or negative) is greater than the voltage in the other direction so that the area under the positive portion of the curve does not equal the area under the negative portion of the curve. This unbalanced waveform may result in a more pronounced treatment effect as the dominant positive or negative amplitude leads to a longer duration of same charge cell membrane charge potential. In this embodiment, the first positive peak 408 has a set voltage 416 (V1) that is larger than the set voltage 416' (V2) of the first negative peak 410. Fig. 7 illustrates further examples of waveforms having unequal voltages. Here, four different types of packets are shown in a single diagram for condensed illustration. The first packet 402 is comprised of pulses having unequal voltages but equal pulse widths, along with no switch times and dead times. Thus, the first packet 402 is comprised of four biphasic pulses, each comprising a positive peak 408 having a first voltage V1 and a negative peak 410 having a second voltage V2). Here the first voltage V1 is greater than the second voltage V2. The second packet 404 is comprised of pulses having unequal voltages but symmetric pulse widths (as in the first pulse 402), with switch times equal to dead times. The third packet 405 is comprised of pulses having unequal voltages but symmetric pulse widths (as in the first pulse 402), with switch times that are shorter than dead times. The fourth packet 407 is comprised of pulses having unequal voltages but symmetric pulse widths (as in the first pulse 402), with switch times that are greater than dead times. It may be appreciated that in some embodiments, the positive and negative phases of biphasic waveform are not identical, but are balanced, where the voltage in one direction (i.e., positive or negative), is greater than the voltage in the other direction but the length of the pulse is calculated such that the area under the curve of the positive phase equals the area under the curve of the negative phase.

In some embodiments, imbalance includes pulses having pulse widths of unequal duration. In some embodiments, the biphasic waveform is unbalanced, such that the voltage in one direction is equal to the voltage in the other direction, but the duration of one direction (i.e., positive or negative) is greater than the duration of the other direction, so that the area under the curve of the positive portion of the waveform does not equal the area under the negative portion of the waveform.

Fig. 8 illustrates further examples of waveforms having unequal pulse widths. Here, four different types of packets are shown in a single diagram for condensed illustration. The first packet 402 is comprised of pulses having equal voltages but unequal pulse widths, along with no switch times and dead times. Thus, the first packet 402 is comprised of four biphasic pulses, each comprising a positive peak 408 having a first pulse width PW1 and a negative peak 410 having a second pulse width PW2). Here the first pulse width PW1 is greater than the second pulse width PW2. The second packet 404 is comprised of pulses having equal voltages but unequal pulse widths (as in the first pulse 402), with switch times equal to dead times. The third packet 405 is comprised of pulses having equal voltages but unequal pulse widths (as in the first pulse 402), with switch times that are shorter than dead times. The fourth packet 407 is comprised of pulses having equal voltages but unequal pulse widths (as in the first pulse 402), with switch times that are greater than dead times.

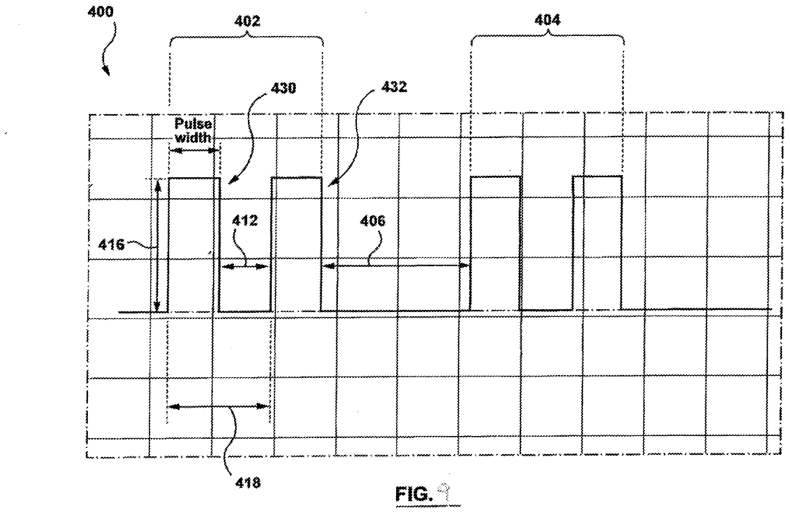

Fig. 9 illustrates an example waveform 400 prescribed by another energy delivery algorithm 152 wherein the waveform is monophasic, a special case of imbalance whereby there is only a positive or only a negative portion of the waveform. Here, two packets are shown, a first packet 402 and a second packet 404, wherein the packets 402, 404 are separated by a rest period 406. In this embodiment, each packet 402, 404 is comprised of a first monophasic pulse 430 and a second monophasic pulse 432. The first and second monophasic pulses 430, 432 are separated by dead time 412 between each pulse. This monophasic waveform could lead to a more desirable treatment effect as the same charge cell membrane potential is maintain for longer durations. However, adjacent muscle groups will be more stimulated by the monophasic waveform, compared to a biphasic waveform.

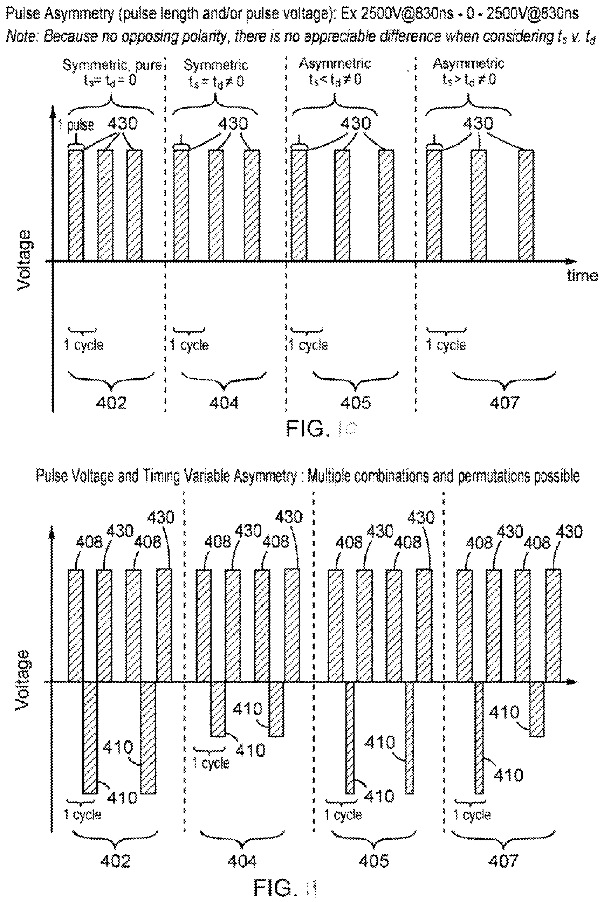

Fig. 10 illustrates further examples of waveforms having monophasic pulses. Here, four different types of packets are shown in a single diagram for condensed illustration. The first packet 402 is comprised of pulses having identical voltages and pulse widths, with no switch times (because the pulses are monophasic) and a dead time equal to the active time. In some cases, there may be less dead time duration than the active time of a given pulse. Thus, the first packet 402 is comprised of three monophasic pulses 430, each comprising a positive peak. In instances where the dead time is equal to the active time, the waveform may be considered unbalanced with a fundamental frequency representing a cycle period of 2x the active time and no dead time. The second packet 404 is comprised of monophasic pulses 430 having equal voltages and pulse widths (as in the first packet 402), with larger dead times. The third packet 405 is comprised of monophasic pulses 430 having equal voltages and pulse widths (as in the first packet 402), and even larger dead times. The fourth packet 407 is comprised of monophasic pulses 430 having equal voltages and pulse widths (as in the first packet 402), with yet larger dead times.

In some embodiments, an unbalanced waveform is achieved by delivering more than one pulse in one polarity before reversing to an unequal number of pulses in the opposite polarity. Fig. 11 illustrates further examples of waveforms having such phase imbalances. Here, four different types of packets are shown in a single diagram for condensed illustration. The first packet 402 is comprised of four cycles having equal voltages and pulse widths, however, opposite polarity pulses are intermixed with monophasic pulses. Thus, the first cycle comprises a positive peak 408 and a negative peak 410. The second cycle is monophasic, comprising a single positive pulse with no subsequent negative pulse 430. This then repeats. The second packet 404 is comprised of intermixed biphasic and monophasic cycles (as in the first packet 402), however the pulses have unequal voltages. The third packet 405 is comprised of intermixed biphasic and monophasic cycles (as in the first packet 402), however the pulses have unequal pulse widths. The fourth packet 407 is comprised of intermixed biphasic and monophasic pulses (as in the first packet 402), however the pulses have unequal voltages and unequal pulse widths. Thus, multiple combinations and permutations are possible.

H. Waveform Shapes

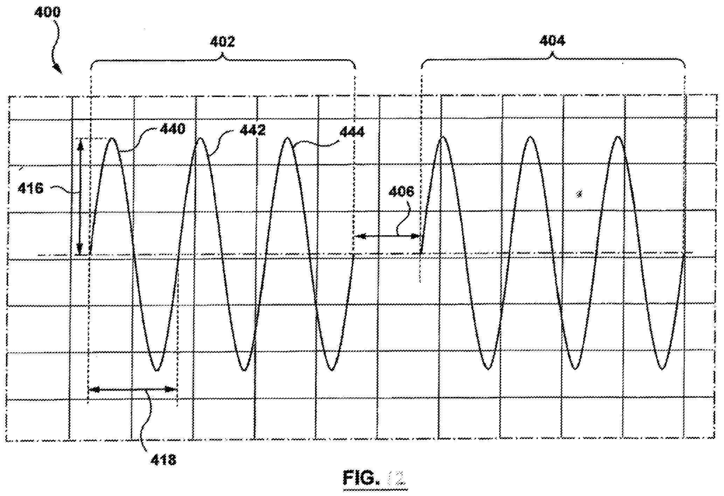

Fig. 12 illustrates an example waveform 400 prescribed by another energy delivery algorithm 152 wherein the pulses are sinusoidal in shape rather than square. Again, two packets are shown, a first packet 402 and a second packet 404, wherein the packets 402, 404 are separated by a rest period 406. In this embodiment, each packet 402, 404 is comprised three biphasic pulses 440, 442, 444. And, rather than square waves, these pulses 440, 442, 444 are sinusoidal in shape. One benefit of a sinusoidal shape is that it is balanced or symmetrical, whereby each phase is equal in shape. Balancing may assist in reducing undesired muscle stimulation. It may be appreciated that in other embodiments the pulses have decay-shaped waveforms.

Energy delivery may be actuated by a variety of mechanisms, such as with the use of a button 164 on the catheter 102 or a foot switch 168 operatively connected to the generator 104. Such actuation typically provides a single energy dose. The energy dose is defined by the number of packets delivered and the voltage of the packets. Each energy dose delivered to the tissue maintains the temperature at or in the tissue below a threshold for thermal ablation. In addition, the doses may be titrated or moderated over time so as to further reduce or eliminate thermal build up during the treatment procedure. Instead of inducing thermal damage, defined as protein coagulation at sites of danger to therapy, the energy dose provide energy at a level which induces treats the condition without damaging sensitive tissues.

Use of Conventional Ablation Catheters

In some situations, it may be desired to utilize a conventional ablation catheter in the tissue modification system 100 described herein. With devices, systems and methods described herein, such conventional ablation catheters may be used in place of catheter 102 to deliver the high voltage pulsed electric fields described herein, either alone or in combination with delivery of other energy, such as energy for conventional ablation. Example conventional ablation catheters include radiofrequency catheters typically used to treat atrial fibrillation, radiofrequency catheters typically used to treat other cardiac arrhythmias, microwave catheters and others. Examples include but are not limited to:

1) Catheters and devices by Abbott Laboratories (Chicago, IL), including Livewire™ TC Ablation Catheter, Safire™ Ablation Catheter, Safire™ TX Ablation Catheter, Therapy™ Ablation Catheter, FlexAbility™ Ablation Catheter, Sensor Enabled™, FlexAbility™ Irrigated Ablation Catheter, TactiCath™ Contact Force Irrigated Ablation Catheter, Sensor Enabled™, TactiCath™ Quartz Contact Force Ablation Catheter, Therapy™ Cool Path™ Ablation Catheter;

2) Catheters and devices by Biosense Webster Inc. (Irvine, CA) including THERMOCOOL® SMARTTOUCH® SF Uni-Directional Catheter, THERMOCOOL® SMARTTOUCH® SF Bi-Directional Catheter, THERMOCOOL® SMARTTOUCH® Uni-Directional Catheter, THERMOCOOL® SMARTTOUCH® Bi-Directional Catheter, THERMOCOOL® SF NAV Uni-Directional Catheter, THERMOCOOL® SF NAV Bi-Directional Catheter, THERMOCOOL® SF NAV Uni-Directional Catheter with curve visualization, THERMOCOOL® SF NAV Bi-Directional Catheter with curve visualization, NAVISTAR® THERMOCOOL® Uni-Directional Catheter, NAVISTAR® THERMOCOOL® Bi-Directional Catheter, NAVISTAR® 4 mm Catheter, NAVISTAR® DS Catheter, NAVISTAR® RMT THERMOCOOL® Catheter, NAVISTAR® RMT 4 mm Catheter, THERMOCOOL® SF Uni-Directional Catheter, THERMOCOOL® SF Bi-Directional Catheter, EZ STEER® THERMOCOOL® Catheter, EZ STEER® 4 mm Bi-Directional Catheter, EZ STEER® DS Bi-Directional Catheter, CELSIUS® THERMOCOOL® Uni-Directional Catheter, CELSIUS® RMT THERMOCOOL® Catheter, CELSIUS® 4 mm Catheter Thermocouple, CELSIUS® 4 mm Catheter Thermistor, CELSIUS® 4 mm Braided Tip Catheter, CELSIUS FLTR® 8 mm Uni-Directional Catheter, CELSIUS FLTR® 8 mm Bi-Directional Catheter, CELSIUS® DS Catheter, CELSIUS® RMT Catheter;

3) Catheters and devices by Boston Scientific Corporation (Marlborough, MA) and/or BARD EP including BLAZER PRIME™ Temperature Ablation Catheter, BLAZER™ II Temperature Ablation Catheter Family, BLAZER™ Open Irrigated Temperature Ablation Catheter, INTELLANAV™ XP & INTELLANAV MIFI™ XP Temperature Ablation Catheter Family, INTELLANAV™ ST Ablation Catheter, INTELLANAV™ OPEN-IRRIGATED Ablation Catheter, INTELLATIP MIFI™ XP Temperature Ablation Catheter, INTELLATIP MIFI™ OPEN-IRRIGATED Ablation Catheter, INTELLANAV™ ST Ablation Catheter,

4) Catheters and devices by Medtronic Inc. (Fridley, Minnesota) including 7Fr RF Marinr™ MC Catheter, 5 Fr RF Marinr™ Catheter, RF Contactr™ Catheter, RF Enhancr™ II Catheter, RF Conductr™ MC Catheter;

5) Catheters and devices by Access Point Technologies EP, Inc. (Rogers, MN) including EP Map-iT™ Catheter, Map-iTTM Irrigation Ablation Catheter;

6) Catheters and devices by Synaptic Medical, Inc. (Lake Forest, CA) including Rithm Cool™ Irrigated Tip Ablation Catheter, Rithm Rx® Deflectable Ablation Catheter, AquaSense® Micro Infusion Irrigated Tip Ablation Catheter;

7) Catheters and devices by Osypka Medical GmbH (Berlin, Germany)/ Cardiotronic - Osypka Medical, Inc. (La Jolla, CA) including Cerablate® easy / Cerablate® easy TC, Cerablate cool®, Cerablate flutter®;

8) Catheters and devices by Biotronik GmbH & Co. (Berlin, Germany) and/or Acutus Medical Inc. (Carlsbad, CA) including AlCath Gold FullCircle, AlCath Flutter Gold, AlCath Flux eXtra Gold;

9) Catheters and devices by Atricure, Inc. (Mason, OH) including Isolator Synergy Clamps, Isolator Synergy Access Clamp, COBRA Fusion 150 Ablation System, Coolrail Linear Pen, Isolator Linear Pen, Isolator Transpolar Pen

10) Catheters and devices by OSCOR, Inc. (Palm Harbor, FL), etc.

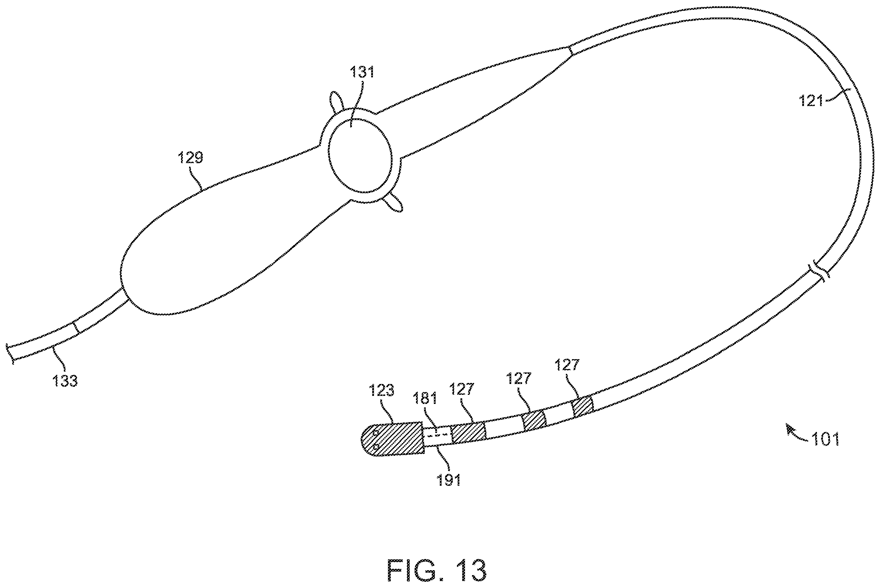

However, these conventional ablation catheters are not configured to deliver the high voltage biphasic PEF energy described herein. In particular, many of these conventional catheters have features and mechanisms that fail under the conditions of high voltage energy delivery. Such failures disable these features and mechanisms, and potentially lead to failure of the device overall. For example, many conventional ablation catheters have a plurality of electrodes near its distal tip. Fig. 13 illustrates an embodiment of a conventional ablation catheter 101 comprising a shaft 121 having a distal tip electrode 123 and a plurality of ring electrodes 127 near its distal end, proximal to the distal tip electrode 123. In this embodiment, the catheter 101 includes a contact force sensor 181 located proximal to the distal tip electrode 123 and three internal fiber optic cables within the shaft 121. Further, the catheter 101 includes an electromagnetic sensor 191 located proximal to the distal tip electrode for integration with a cardiac mapping system. In addition, the catheter 101 has a handle 129 disposed near the proximal end of the shaft 121. In this embodiment, the handle 129 includes a universal actuator design 131 which allows for deflection, independent of handle position, and tension locking which allows for variable control. The handle 129 has an integrated cable 133 for connection, such as to the generator 108.

Since each of the electrodes within a catheter 101 are commonly intended to be independently activated, each of the electrodes 123, 127 have their own conductive wire extending through the catheter to its proximal end. These conductive wires are contained within the body of the catheter and, typically, are each surrounded by an insulative layer to avoid undesired short circuits between conductors.

However, when delivering the pulsed electric field energy described herein, these conventional ablation catheters 101 are often prone to arcing and shorting. This is caused by the high voltage energy delivered through the various conductors within the conventional catheters. Since the conventional ablation catheters 101 are designed for lower voltage, the conduction wires are not arranged to insulate the wires from each other and the insulation material that is utilized is insufficient to properly insulate the wires under these conditions. Consequently, the insulation material fails allowing the wires to short together and generate arcing within the catheter body. All of these issues make such use undesired or impossible for high voltage energy delivery and for switching between high voltage energy delivery and conventional energy (e.g. radiofrequency, microwave, etc).

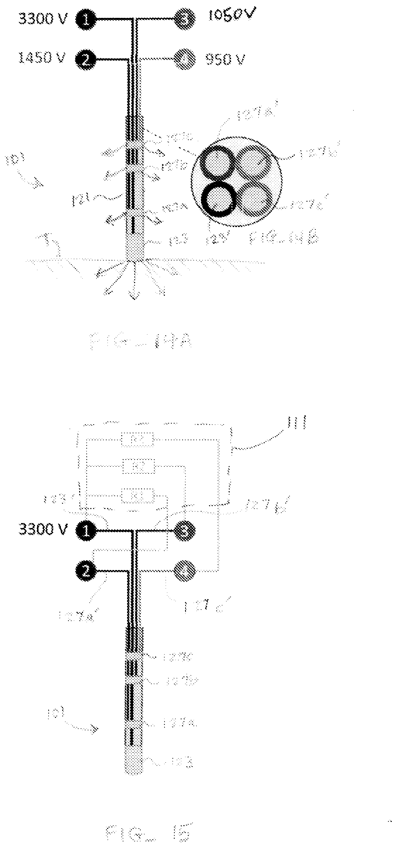

Fig. 14A illustrates some of the issues related to high voltage energy delivery through a conventional ablation catheter having a plurality of electrodes, such as the catheter 101 depicted in Fig. 13. Here, the distal end of a catheter 101 is shown having an elongate shaft 121 with a delivery electrode 123 at its tip. In this embodiment, the catheter 101 includes three additional ring electrodes: a secondary electrode 127a, a tertiary electrode 127b and a quaternary electrode 127c, each spaced an incremental distance proximally along the shaft 121 from the delivery electrode 123. During use, the delivery electrode 123 is positioned against the target tissue T, such as cardiac tissue. Energy delivered through the delivery electrode 123 enters the tissue T as shown. However, when treating cardiac tissue, the environment is typically blood filled. Due to the conductivity of blood, energy is also transmitted through the conductive wires leading to the secondary electrode 127a, tertiary electrode 127b and quaternary electrode 127c as indicated by arrows in Fig. 14A.

From an active electrode (at voltage $V = V_0$) to a ground electrode ($V = 0$) the voltage will decrease as the current (J) cross the medium (blood or tissue) according to its electric conductivity ($\sigma$).

$$-\nabla V = \boldsymbol{J}\sigma$$

This degradation of the applied voltage over the whole medium results in a spatial distribution of potential. As the unused electrodes are in contact to some parts of the medium a defined voltage in those can be expected.

The expected voltage in each of the electrodes can be obtained using numerical approaches. Analytic solutions are only feasible when simple geometries and homogeneous conductivities are employed. However, since both tissue and electrode geometries can be complex, it is more likely to determine the voltage distribution using finite elements method and solving the electric potential (V) that satisfies the following Laplace equation.

$$\nabla \cdot (\sigma \nabla V) = 0$$

Using this method, one can compute the voltage distribution for the desired catheter geometry and environment.

Using this method, the voltages of the delivery electrode 123, secondary electrode 127a, tertiary electrode 127b and quaternary electrode 127c can be determined. The delivery electrode 123 has the maximum voltage and the voltage values decrease with distance from the delivery electrode 123. Since all the electrodes are connected to the internal conduction wires, from the computed values one can extract the voltage in each of those, thus, determine the maximum voltage difference between them. For example, if the energy delivered to the delivery electrode 123 has a voltage of 3300V, the energy transmitted to the secondary electrode 127a would have a voltage of 1450V, the energy transmitted to the tertiary electrode 127b would have a voltage of 1050V, and the energy transmitted to the quaternary electrode 127c would have a voltage of 950V. This poses a variety of issues. To begin, each of the electrodes 123, 127a, 127b, 127c are connected to the proximal end of the catheter 101 by insulated conduction wires. Fig. 14B illustrates a cross-section of shaft 121 showing the insulated conduction wires 123', 127a', 127b', 127c' corresponding the electrodes 123, 127a, 127b, 127c. In conventional ablation catheters, such insulation is not sufficient to insulate beyond a voltage differential of approximately 1500V (although this value may vary depending on the specific design of the catheter). In the example illustrated in Fig. 14A, the differential between the delivery electrode 123 and the secondary electrode 127a is 1850V, the differential between the delivery electrode 123 and the tertiary electrode 127b is 2250V and the differential between the delivery electrode 123 and the quaternary electrode 127c is 2350V. Each of these exceed the 1500V threshold causing insulation failure which leads to shorting between the conduction wires and arcing.

Voltages can be brought below the threshold level for shorting and/or arcing by a variety of systems and methods. For example, in some embodiments, each of the electrodes 123, 127a, 127b, 127c are set to the same voltage. Since each of the conduction wires will have the same potential, the voltage difference between conduction wires is null and arcing will not occur. However, this straightforward solution will have several inconveniences. First, by activating all of the electrodes 123, 127a, 127b, 127c in this manner, each will deliver the treatment energy thereby possibly delivering the energy to undesired areas. Second, for the same applied voltage, the total current injected in the body is higher which may result in an excessive increase in temperature or muscle stimulation. In addition, such high current demands require a pulse generator with increased performance.

In other embodiments, a component network 111, such as comprised of passive components (e.g. resistors, inductors and diodes), are used to modulate the energy flowing from the pulse generator to the electrodes. The passive components combine to form a complex impedance, Z, that acts to steer the energy through the conduction wires in a predetermined fashion so that the voltage differentials stay below a particular threshold level, such as 1500V. In some embodiments, the component network 111 is disposed within, for example, the generator 108 to which the catheter 101 is coupled for energy delivery, disposed within a separate device in line with the generator 108 (e.g. within an interface connector 10), or within the catheter 101 or an accessory to the catheter 101.

The resistor, capacitor, and inductor values for the impedances may vary depending on a variety of factors, including the frequency and amplitude of the applied electric energy. For example, the impedance of an inductor is directly proportional to applied frequency, while the impedance of a capacitor is inversely proportional to the applied frequency. For a given set of applied energy parameters, such as voltage, amplitude and frequency, the complex impedance can be predetermined to modulate the energy flowing from the pulse generator to the electrodes.

In one embodiment, schematically illustrated in Fig. 15, a first resistor R1 is positioned between conduction wire 123' (coupled to delivery electrode 14) and conduction wire 127a' (coupled to secondary electrode 127a). Likewise, a second resistor R2 is positioned between conduction wire 123' (coupled to delivery electrode 123) and conduction wire 127b' (coupled to tertiary electrode 127b). Further, a third resistor R3 is positioned between conduction wire 123' (coupled to delivery electrode 127) and conduction wire 127c' (coupled to quaternary electrode 127c).

The resistor values for the resistors R1, R2, R3 may vary depending on a variety of factors, including the geometry and relative position of the electrodes and the electric conductivity of the surrounding medium. For example, the larger the distance between the active electrode and a non-active electrode, the lower the induced voltage will be in the non-active electrode when a voltage is applied over the active electrode. When desiring to preserve the voltage difference between the active and non-active electrodes under a certain value, the larger distances between particular electrodes will involve increasing a bit more the induced voltage in the corresponding non-active electrode to stay within the constraint of the maximum allowable voltage differential. In embodiments having a single non-active electrode, one can tune the proposed resistor until the desired voltage is generated at this non-active electrode. However, in embodiments having more than one non-active electrode, decreasing the value of one resistor will increase the current flowing through that non-active electrode and the induced voltage, but will also increase the voltage at the other non-active electrodes. This inter-dependence between the resulting electrode voltages and the resistor values entails a highly complex system.

This complexity is addressed with a tridimensional mathematical model of the electrodes and the potential environment. The model takes into account the different elements expected in the environment when treating cardiac tissue, particularly tissue surrounded by blood. Therefore, the properties of these elements are specified and will determine the voltage distribution when a voltage is applied.

The mathematical model defines the voltage at the surface of the active electrode ($V = V_0$), and a current source at the non-active electrodes ($I_x$) is defined as a current source (integral of the normal current density $J$ along its surface $S$) with dependence on the voltage at the electrode surface, the applied voltage at the active electrode and the selected resistor value.

$$\int_{\partial\Omega} \boldsymbol{J} \cdot \boldsymbol{n}\, dS = I_x = \frac{V_0 - V}{R_x}$$

With this model, the potential combinations of resistor values (in a defined range and resolution) are determined. The final values are selected that show the desired performance. In the embodiment depicted in Fig. 15, wherein the catheter 101 has four electrodes (one active and 3 non-active) the combination of the three resistor values (R1, R2, R3) are computed (e.g. from 0 to 1000 in steps of 10 Ω) to preserve the voltage difference under a maximum voltage difference supported by the conduction wires/insulation (e.g. 1500V) and a total current safety value for the pulse generator (e.g. under 40 A).

In this example, the energy delivered through conduction wire 123' to the delivery electrode 123 is 3500V. Likewise, in this embodiment, the total current has a maximum safety value of 40A. Consequently, in this embodiment, the total resistance network combination has a maximum of 1000 to 1200 ohms. For example, in one embodiment the resistor values are as follows: R1= 500 Ω, R2= 300 Ω and R3= 300 Ω. Using these resistor values, the voltage differential between the conduction wires are as shown in Table 1.

Table 1.

| Conduction wire to | Voltage (V) | Voltage differential from delivery electrode conduction wire (V) |
| --- | --- | --- |
| Delivery electrode 123 | 3500 | N/A |
| Secondary electrode 127a | 2177 | 1323 |
| Tertiary electrode 127b | 2127 | 1373 |
| Quaternary electrode 127c | 2061 | 1439 |

Consequently, the network of resistors is able to keep the voltage differentials below the threshold for shorting and arcing (e.g. 1500V) while maintaining the desired high voltage energy delivery to the delivery electrode 123 (e.g. 3500V). It may be appreciated that, in this example, the current through each of the conduction wires are as shown in Table 2 and total approximately 40 amps.

Table 2.

| Conduction wire to | Current (A) |
| --- | --- |
| Delivery electrode 123 | 27.9 |
| Secondary electrode 127a | 2.6 |
| Tertiary electrode 127b | 4.6 |
| Quaternary electrode 127c | 4.8 |
| Total | 39.9 |

In some instances, the use of the network of resistors results in the creation of a slightly smaller lesion (e.g. up to 30% smaller) in the target tissue, thereby potentially reducing the efficacy of the treatment. However, this can be compensated by increasing the treatment intensity (i.e. voltage). This is possible because the network of resistors also increases the threshold for arcing and shorting. In some instances, the resistor network reduces the maximum current density by 20%. Thus, the applied voltage may be increased by the same magnitude to compensate for this difference. Similarly, when employing the network of resistors, other waveform characteristics may also be increased to adjust for treatment intensity, such as increasing cycle counts, decreasing the fundamental frequency of the waveform, integrating varying degrees of asymmetry to the waveform, or adding additional packets.

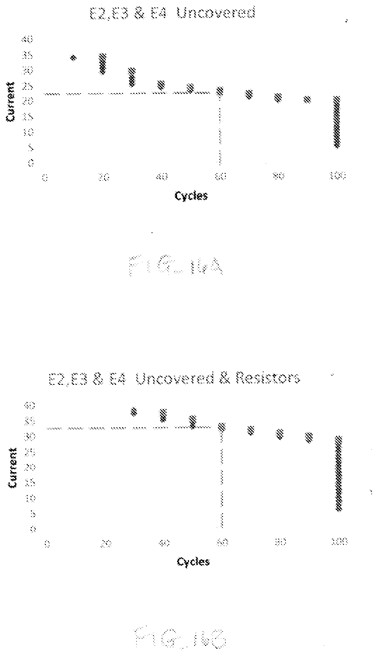

Figs. 16A-16B illustrate the increase in threshold for arcing when using the component network 111 of Fig. 15. Fig. 16A corresponds to the situation in which no component network 111 is utilized. The graph illustrates the current values in which arcing occurred over a series of pulse cycles. In particular, a quantity of energy (e.g. 100 cycles) was delivered at a particular current value and the presence or absence of arcing was observed. If no arcing was observed, the current value was increased. This continued until arcing was observed. Therefore, the highest value along a cycle vertex (e.g. the 100 cycle vertex) is the arcing threshold. Thus, as shown in Fig. 16A, when 60 cycles of energy were delivered through the catheter 101 without the component network 111, the threshold for arcing occurred at a current value of 22.2 amps or 2100V (this corresponds to the highest data point along the 60 cycle vertex). Fig. 16B corresponds to the situation in which the component network 111 of Fig. 15 is utilized, wherein R1=500, R2=300, R3=300. Here, the graph illustrates the current values in which arcing occurred over a series of pulse cycles which can be seen to be higher than that of Fig. 16A. In particular, at 60 cycles, the current value was 32.7 amps or 2700V. This corresponds to a 47% increase in current threshold for arcing. Therefore, the use of a component network 111 significantly increases the threshold for arcing by preventing uncontrolled current flow through the secondary electrode 127a, tertiary electrode 127b and quaternary electrode 127c.

It may be appreciated that other systems and devices may be used to function in a similar manner as the component network 111. For example, in some embodiments, the generator 108 is configured to send the appropriate current to the conductor wires within the catheter 101 so as to keep the voltage differential between the conductor wires below a threshold for arcing or damage to the catheter 101. In some instances, the generator 108 is configured to be used with a particular catheter, such as a particular conventional radiofrequency catheter listed above. Thus, the electrode spacing and other features of the catheter are known. Consequently, the generator 108 may be pre-programmed or pre-configured to deliver the appropriate energy through each conductor wire appropriate for the particular catheter. For example, when delivering to the catheter 101 illustrated in Fig. 15, a multi-channel generator may be used to drive the voltage of the secondary electrode 127a, tertiary electrode 127b and quaternary electrode 127c to a lower voltage than the distal electrode of the catheter in order to keeps the voltage differential between the conductor wires below a threshold for arcing (e.g. 1500V). This may be accomplished using a multi-channel generator that incorporates two banks of capacitors, a primary bank of capacitors that is charged to drive the delivery of energy to the delivery electrode 123 of the catheter, and a secondary bank of capacitors that is charged to a lower voltage than the primary capacitor bank and drives the delivery of energy to the secondary electrode 127a, tertiary electrode 127b and quaternary electrode 127c. Further, additional banks of capacitors may be used to drive different levels of energy to the secondary electrode 127a, tertiary electrode 127b and quaternary electrode 127c. In each of these situations, the voltage differential between the conductor wires is maintained below a threshold for arcing (e.g. 1500V).

In some embodiments, the generator is configured to be used with a variety of catheters. In some instances, the generator is programmable to be used with particular catheters, wherein the user is able to indicate which catheter is being used. For example, the generator may display a variety of selectable options from a menu corresponding to known catheters or known aspects of typical catheters, such as electrode number, electrode spacing, catheter type, etc. Once the identifying aspects are selected, the generator utilizes pre-programmed algorithms corresponding to each type of known catheter or known set of features (e.g. electrode arrangement, etc.) to send the appropriate current to the conductor wires within the catheter so as to keep the voltage differential between the conductor wires below a threshold for arcing or damage to the catheter. In other embodiments, aspects of the catheter are identified by the generator. For example, in some instances, the catheter is connectable to the generator wherein the generator is able to measure, sense or identify aspects of the catheter which indicate the appropriate energy to be delivered through each of the conduction wires. In some embodiments, the generator delivers a dose of low voltage energy through each of the conductor wires so as to measure its corresponding impedance. The generator then delivers the appropriate current through each of the conductor wires within the catheter based on the impedance measurements. In some embodiments, the catheter is analyzed by the generator while the catheter is in the treatment environment and/or in position to provide the treatment. Thus, environmental or situational factors that affect the impedance readings are taken into account. This may include the presence of blood or other conductive fluids. Alternatively or in addition, this may include the position or arrangement of the device. For example, some devices may have electrodes along surfaces, such as arms, that may be disposed in various positions. Thus, the distance between various electrodes may vary depending on the positions of these surfaces. By evaluating device while positioned at the target location, these aspects are taken into account. This may result in more precise delivery of current values through the conduction wires during treatment.

It may be appreciated that such variability in environmental conditions may also be accommodated by a component network 111, rather than the generator itself. In such embodiments, the component network 111 may be comprised of one or more potentiometers, rheostats, variable resistors, capacitors, inductors, diodes or the like. In other embodiments, the component network 111 may be comprised of a plurality of resistors which are selectable by a controller as desired. In either case, a desired resistance is applied to each of the conductive wires according to Fig. 15 wherein the R values (R1, R2, R3) are chosen based on measured values, such as impedance values.

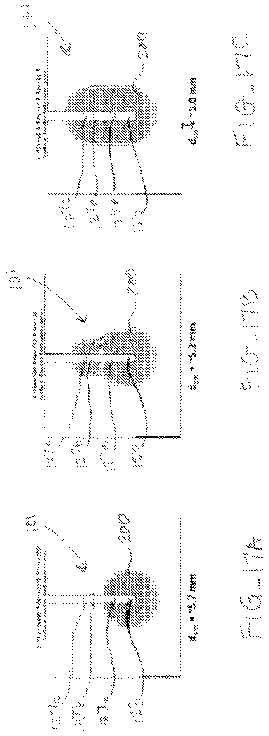

In addition, in some embodiments, the component network 111 and systems described herein is utilized to shape the electric field delivered by the catheter 101. For example, Figs. 17A-17C illustrate various electric fields delivered by the catheter 101 with the use of different resistor values. Figs. 17A-17C illustrates the catheter 101 having a delivery electrode 123, a secondary electrode 127a, a tertiary electrode 127b, and a quaternary electrode 127c near its distal end. In Fig. 17A, the catheter 101 is connected to a component network 111 as in Fig. 16 having R1=10000 Ω, R2=10000 Ω, R3=10000 Ω (equivalent to being not connected to a component network 111). The resulting electric field 200 has a somewhat rounded shape emanating primarily from the delivery electrode 123. Fig. 17B illustrates the same catheter 101 connected to a component network 111 as in Fig. 15 having R1=500 Ω, R2=300 Ω, R3=300 Ω. The resulting electric field 200 has now shifted to a pear shape having a somewhat circular shape emanating from the delivery electrode 123 and secondary electrode 127a along with a smaller, overlapping somewhat circular shape emanating from the tertiary electrode 127b and quaternary electrode 127c to create the pear shape. Fig. 17C illustrates the same catheter 101 where all the electrodes are active. The resulting electric field 200 has now shifted to an oblong or elliptical shape extending from electrodes 123, 127a, 127b, 127c. Thus, a desired electric field 200 shape may be generated by choosing the appropriate the resistor values. This also affects the dimensions of the resultant lesion, wherein the depth and width are dependent on the shape of the electric field. 200.

As indicated above, it may be appreciated that component networks 111 and systems described herein may be used with catheters 101 having differing numbers of electrodes than the example provided herein, such as two electrodes, three electrodes, four electrodes, five electrodes, six electrodes, seven electrodes, eight electrodes, nine electrodes, ten electrodes, 2-4 electrodes, 2-5 electrodes, 2-10 electrodes, 10-15 electrodes, 2-20 electrodes, 2-30 electrodes, 2-40 electrodes, 2-50 electrodes, 2-60 electrodes, 2-70 electrodes, 2-80 electrodes, 2-90 electrodes, 2-100 electrodes and over 100 electrodes. Likewise, the component networks 111 and systems described herein may be used with catheters 101 having arrangements of electrodes other than the example provided herein, such as having different spacing between the electrodes and having the electrodes aligned non-linearly, such as around a ring, or on branching splines, each containing one or more electrodes, etc. It may also be appreciated that separate electrodes may act as a single electrode if they adjacent or sufficiently near each other and are electrified simultaneously. In such instances, the separate electrodes are counted as a single electrode and behave as a single electrode in the examples provided herein.

It may also be appreciated that the component networks 111 and systems described herein may also be used with catheters designed for bipolar energy delivery. Thus, the PEF energy described herein may be delivered to a target tissue with the use of a bipolar energy delivery catheter used in a monopolar fashion. In such instances, one of the electrodes on the bipolar energy delivery catheter is utilized as the delivery electrode and the remaining electrodes are considered additional electrodes (i.e. secondary electrode, tertiary electrode, quaternary electrode, etc.). Thus, a component network 111 or system based on the same principles as described above may be used with a bipolar energy delivery catheter. Example bipolar energy delivery catheters are those provided by Farapulse (Menlo Park, CA), Affera, Inc. (Watertown, MA), Atrian Medical (Galaway, Ireland), Kardium, Inc. (Burnaby, BC, Canada), to name a few.

It may be appreciated that the component 111 networks and systems described herein may be used with any high voltage energy, including high frequency irreversible electroporation, pulsed radiofrequency ablation, nanosecond pulsed electric fields, etc. Other example high voltage energy is described in US Publication No. 20190201089, entitled METHODS, APPARATUSES, AND SYSTEMS FOR THE TREATMENT OF PULMONARY DISORDERS, filed December 20, 2018; described in WO/2019/133606, entitled METHODS, APPARATUSES, AND SYSTEMS FOR THE TREATMENT OF DISEASE STATES AND DISORDERS, filed December 26, 2018; and described in WO/2019/133608, entitled OPTIMIZATION OF ENERGY DELIVERY FOR VARIOUS APPLICATIONS, filed on December 26, 2018, to name a few.

It may be appreciated that reference to treatment catheters herein typically apply to specialized catheters 102 which are configured to deliver the PEF energy described herein or conventional catheters 101 which have been adapted to deliver the PEF energy described here, such as with the use of one or more accessories. Typically, such treatment catheters are referred to as treatment catheters 102 for ease of readability, however it may be appreciated that such description applies to treatment catheters 101 in many or all occasions.

Tissue Lesions

When treating a variety of cardiac conditions, a range of different target tissue thicknesses are encountered in patients. Therefore, tissue lesions of various depths may be desired. In some embodiments, there is a highly repeatable and clear monotonic trend of lesion size as a result of dose intensity for a single-parameter manipulation. Thus, in some embodiments, when changing a single parameter of the energy, the resultant lesion size is proportionally changed. However, it may be appreciated that the correlation between energy delivered and lesion depth may vary depending on a variety of conditions, such as size, shape and configuration of electrode arrangements along with parameter values and characteristics of the energy waveform, to name a few. Thus, in some embodiments the correlation is non-linear but follows a curve. For a given condition, a variety of optimally titrated doses may be available (e.g. via algorithms 152) for treating the range of expected target tissue thicknesses in patients. Aspects considered for determining the final dose range include cross-sectional width and depth of the target tissue, risk of bubble formation, desired time for treatment delivery, potential temperature rise, ECG waveform and rhythm preservation, safety to phrenic nerve and esophageal tissues, and qualitative safety of resulting treatment effect to the heart itself.

Example doses and their resulting effects are summarized in Table 3.

**Table 3.** Treatment Dose Characterization and Resulting Lesion Characteristics

| Dose | Peak Current | Frequency | Treatment Active time | Number of Treatments | Total Energy Delivered | Lesion Depth, mm |
|------|------|------|------|------|------|------|
| A | 20A | High | 80 us | 2 | 24 J | 2.99 mm |
| B | 15 A | Low | 150 us | 1 | 17 J | 3.09 mm |
| C | 25 A | Low | 117 us | 3 | 73 J | 5.03 mm |
| D | 30 A | High | 90 us | 4 | 122 J | 5.01 mm |
| E | 28 A | Low | 150 us | 6 | 265 J | 7.08 mm |
| F | 36 A | High | 90 us | 10 | 437 J | 7.12 mm |

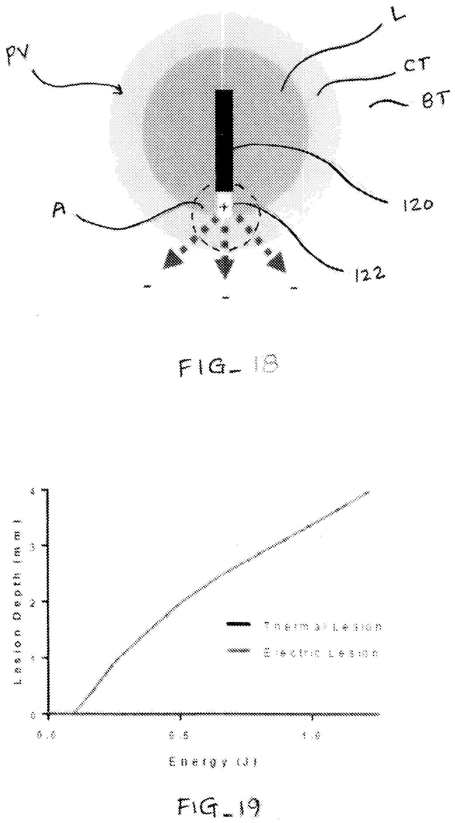

Fig. 18 provides a schematic illustration of a treatment catheter 102 positioned for pulmonary vein isolation. In particular, Fig. 18 illustrates of a cross-section of a lumen L of a pulmonary vein PV surrounded by cardiac tissue CT and then body tissue BT therearound. In this illustration, the lumen L has a diameter of 25 mm and the cardiac tissue CT has a thickness of 4mm. A treatment catheter 102 is shown having a delivery electrode 122 at its distal end; the delivery electrode 122 is illustrated as contacting the cardiac tissue CT via the lumen L. In this embodiment, the electrode 122 delivers the energy in a monopolar fashion wherein the energy flows from the electrode 122 outwardly toward the surface of the body tissue BT (e.g. skin) and the return electrode (not shown) positioned thereon. This electric field creates a treatment area A of varying depth depending on the energy delivery algorithm 152. In this example, a treatment area A penetrating the thickness of 4mm is achieved. It may be appreciated that typically as the energy is increased, the size of the treatment area A likewise increases. An example of the association of energy and treatment area depth is illustrated in the graph of Fig. 19 (sloping line) wherein a lesion of 4mm is able to be achieved with an energy output of approximately 1.5 joules. This is achieved with the use of a focal catheter as illustrated in Fig. 18 and an algorithm 152 producing an energy waveform. It may be appreciated that lesion depths of greater than 4mm, such as 5mm, 6mm, 7mm, 8mm, 9mm, 10mm and greater than 10mm may be achieved with the devices and methods provided herein. Fig. 19 also illustrates the association between energy and thermal effects which is a flat line across the x-axis. Thus, the energy delivered is non-thermal. As mentioned previously, the therapeutic energy delivered through the delivery electrode 122 is generally characterized by high voltage pulses which allow for removal of target tissue with little or no destruction of critical anatomy, such as tissue-level architectural proteins among extracellular matrices. This prevents dangerous collateral effects, such as stenosis and thrombus formation, to name a few.

Particular characteristics of the devices and energy waveforms provided herein provide superior lesion depth to energy usage correlations. Thus, the devices and systems described herein are able to provide deeper lesions with the use of less energy than other known PEF devices using known PEF energy. Less energy correlates to lower thermal effects and reduced demands on the generator. In some embodiments, this is a result of the nature of the electric current distribution. By delivering the energy in a monopolar fashion, the energy is able to penetrate deeper into the cardiac tissue CT than if the energy were delivered in a bipolar fashion. In the monopolar manner, the electric current travels through directly into the tissue toward a remote return electrode, extending deep through the myocardium. This is in contrast to a bipolar pair of electrodes wherein the energy is travelling shallowly into the tissue only to return back to the end effector having the return electrode. Thus, the energy does not travel as deeply. The electric current will follow the path of least resistance, which is directed across the tissue, without much current traveling deep through the tissue. Therefore, for bipolar electrode arrangements, significantly more intense treatment protocols are required to reach deeper treatment depths in the targeted tissue. This characteristic becomes more pronounced as the target depth further increases (i.e., reaching 4mm from 2mm may require ~4x energy, while extending the treatment depth from 2mm to 6mm may require ~16x energy for this design. Depending on the electrode configuration, some bipolar designs require up to 100 joules to achieve the same lesion depth and typically involve a variety of negative side effects such as excessive heating.

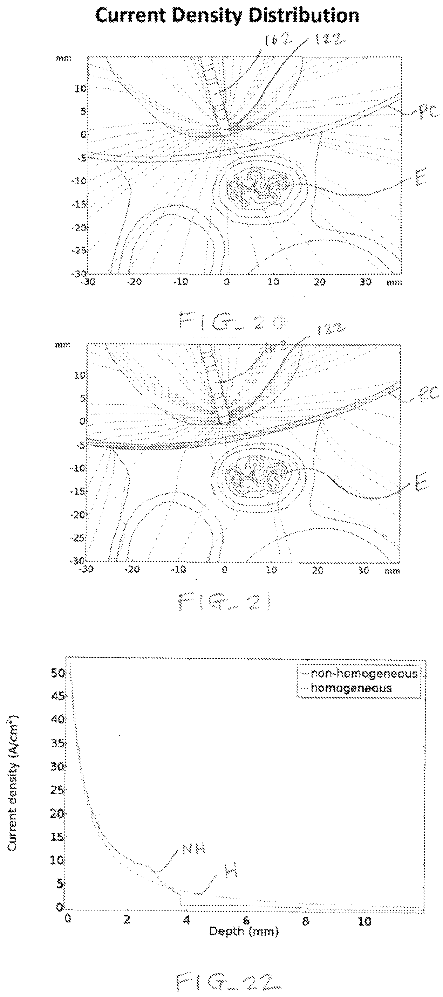

It may be appreciated that although the monopolar PEF energy is able to penetrate more deeply into tissues than either bipolar PEF or RF, nearby critical structures are protected from damage due to the nature of PEF energy and due to the presence of disparate tissue planes in the cardiovascular anatomy. In particular, the pericardial fluid and pericardium surrounding the heart serves to dissipate energy, protecting extracardiac structures, such as the esophagus, phrenic nerve, coronary arteries, lungs, and bronchioles, from injury. This is not the case when delivering energy that creates lesions by thermal injury. In those cases, the propagation of conductive thermal energy beyond the targeted myocardial tissue can result in thermal injury to non-targeted extracardiac structures. Figs. 20-21 illustrate by mathematical modeling the current distribution of PEF energy emanating from a delivery electrode 122 of a catheter 102 (such as illustrated in Fig. 13) under different conditions. Here, the delivery electrode 122 is positioned within the heart near the pericardium PC, beyond which likes the esophagus E. Fig. 20 illustrates the current density distribution when all of the tissues are considered homogenous. Thus, energy lines are shown emanating from the delivery electrode 122 undisturbed, directly into the surrounding tissue. However, Fig. 21 illustrates the current density distribution when the tissues are non-homogenous, taking into account the differences in tissue type for different anatomical structures. As illustrated, the energy lines are shown directed toward the pericardium PC where they are dissipated along the pericardium PC. Consequently, the energy reaching beyond the pericardium PC is dramatically reduced and inconsequential to the surrounding tissues, including the esophagus E. Fig. 22 provides a graphical illustration of current density vs. penetration depth in homogenous vs. non-homogenous tissue. A first curve H corresponds to homogenous tissue wherein the reduction in current density at increasing tissue depths follows a continuous asymptotic curve. A second curve NH corresponds to non-homogenous tissue, as in the modeling provided in Fig. 21, which illustrates the current density changing at various depths according to changes in tissue type.

It may be appreciated that a variety of different types of lesions may be created with the treatment catheters 102 described herein. As mentioned, lesion rings, such as around the outside ostium of a pulmonary vein, can be created with either the focal catheters or the one shot catheters. In addition, the focal catheters can be used to create many other types of lesions, particularly lines along various surfaces of cardiac tissue. In one embodiment, a cavo-tricuspid isthmus line is created for the treatment of typical atrial flutter in the right atrium. In another embodiment, roof lines and/or floor lines are created for a box lesion along the posterior wall of the left atrium for patients with atrial fibrillation, particularly for persistent atrial fibrillation. In another embodiment, a mitral isthmus line is created along the anterior or lateral wall of the left atrium for atypical atrial flutter. In yet another embodiment, ventricular lines are created connecting two inexcitable boundaries that are critical to the initiation or maintenance of a reentrant ventricular arrhythmia, typically in patients with ventricular tachycardia resulting from ischemic heart disease.

Contact and Contact Force

In some embodiments, contact and contact force are assessed to evaluate engagement and ensure uniform electrode to tissue contact. Such assessments are not provided by known PEF devices and systems utilized in treating cardiac tissues, such as in the treatment of atrial fibrillation. It has been asserted that PEF energy delivery simply depends on proximity of an electrode to the target tissue rather than contact. The belief is that the effects of PEF energy on tissue are proximity dependent but not contact dependent because they are a result of the electric field which extends from the electrode. The effects are considered a result of the voltage delivered and the distance over which the voltage is applied. Thus, the effect at any given location within the tissue is dependent on the electric field strength.

However, these known PEF devices and systems rely on bipolar energy delivery which creates an electric field around the electrodes. In contrast, the devices, systems and method described herein are primarily used monopolarly which drives the electric field into the tissue toward a remote return electrode. Lack of contact allows the surrounding blood flow to diverge and disrupt the energy flow, reducing penetration into the tissue. Thus, improved engagement increases the delivery of PEF energy into the tissue. Likewise, uniform engagement optimizes such delivery.

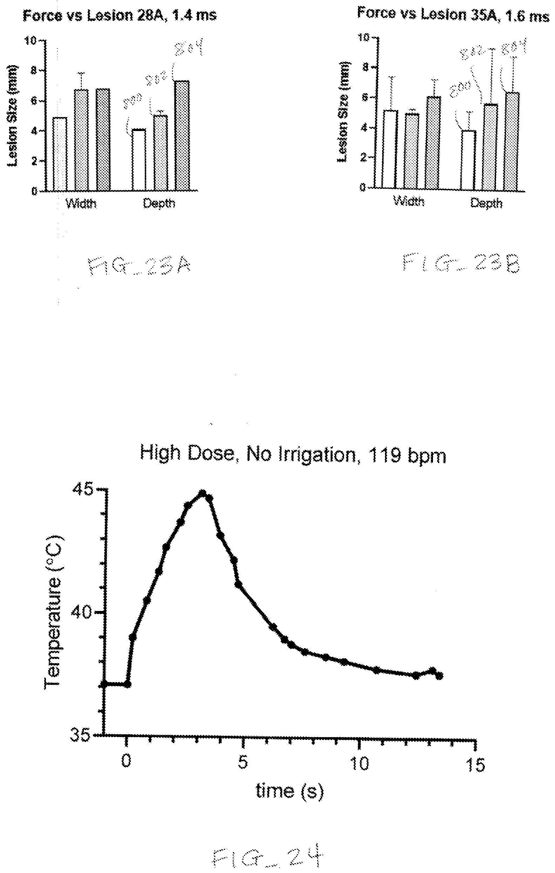

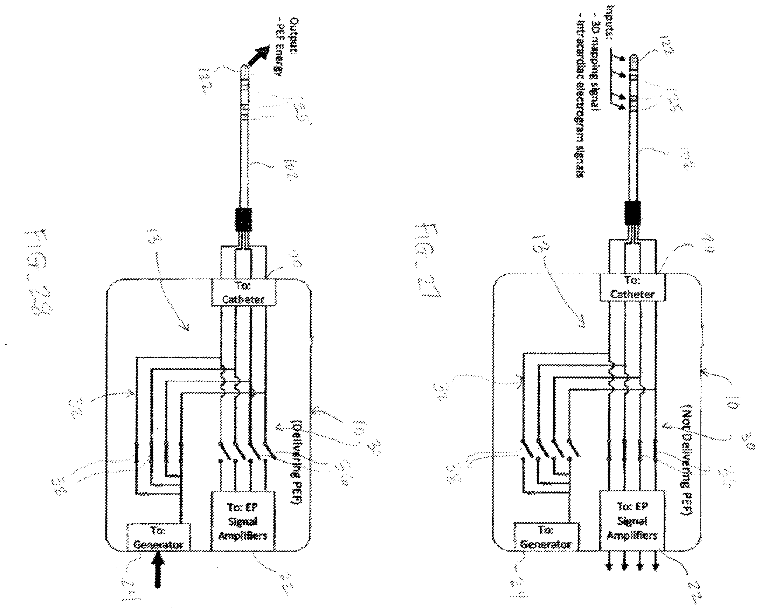

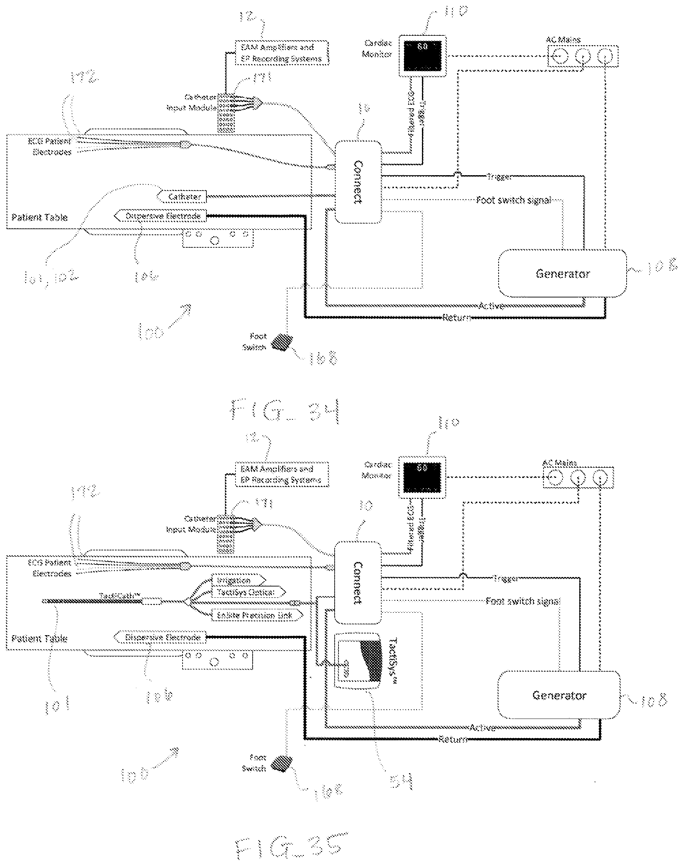

Figs. 23A-23B illustrate the effect of contact force on lesion size, in particular lesion width and depth. Three levels of contact force were evaluated: 1) low contact force 800 (5-15g), 2) medium contact force 802 (15-30g), and 3) high contact force 804 (30 to 50g). Increasing contact force has beneficial effects on both the width and depth of lesion sizes. In Fig. 23A, 28 amps of PEF energy described herein was provided to ventricle tissue in a monopolar configuration for 1.4ms which resulted in lesion depths between approximately 4mm and 8mm. Lesion depth was correlated to contact force, wherein increased contact force resulted in increased lesion depth. In Fig. 23B, 35 amps of PEF energy as described herein was provided to ventricle tissue in a monopolar configuration for 1.6ms which resulted in similar lesion depths between approximately 4mm and 8mm. Again, lesion depth was correlated to contact force, wherein increased contact force resulted in increased lesion depth.

In some embodiments, the treatment catheters described herein include a mechanism to measure contact and/or contact force. In some embodiments, contact is sensed with the use of impedance sensors, particularly impedance between the tip of the catheter 102 and the cardiac tissue. Impedance is represented as a complex number derived from resistance and reactance. In some embodiments, impedance is measured by sensing the impedance characteristics between the electrodes on a focal catheter, between electrodes on the catheter and a separate remote electrode located distantly on the body or other locations within the heart, between electrodes on the catheter and multiple separate electrodes located distantly on the body or other locations within the heart; or by similar combinations with dedicated impedance sensors placed on the tip of the catheter or along the shaft of the electrode where it is desired to determine the presence of electrode contact.

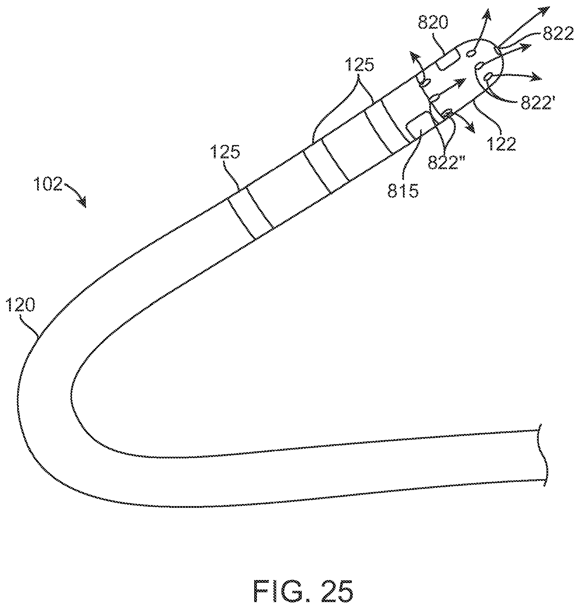

In other embodiments, contact force is sensed. This can be achieved by a number of mechanisms, generalized as a contact force sensor 815 illustrated in Fig. 25. In some embodiments, contact force is measured by detecting the change in a specific wavelength of light reflected in a fiber Bragg grating (FBG). In some embodiments, the contact force sensor 815 comprises an optical sensor with at least one optical fiber is mounted near the distal end of the catheter 102 wherein the distal end of the catheter is deformable. Light is provided through the at least one optical fiber and only the light with the specific wavelength is reflected by the FBG. When contact force is applied to the tip, the tip deforms the sensor body and compression or elongation of the at least one optical fiber changes the periodic cycle of the R refractive index pattern. This change of cycle shifts the wavelength of the reflected light proportionally to the applied force. Thus, direction and magnitude of force is sensed. In some embodiments, the total value and magnitude of axial and lateral component vectors of contact force are provided to the user. In other embodiments, contact force is measured by compression or extension of a spring. In such embodiments, the contact force sensor 815 comprises a small spring is mounted in the distal end of the catheter 102. The degree of spring compression and/or stretching is detected at specific time intervals by at least one receiving sensor located at the base of the spring. The measured contact force values are then provided to the user. In some embodiments, real time values (e.g. direction, force, etc.) are provided in graphical form or any suitable form.

Temperature Sensing and Control

The tissue modification systems 100 described herein deliver a series of PEF batches or bundles described herein over a period of time, such as several seconds. This accumulation of energy deposition results in a small amount of joule heating which is inherent to all PEF therapies as it is a byproduct of energy deposition. While acute, subacute, medium-, and long-term histological data all indicate that there are no substantial indication of thermal damage to the tissue using the systems, devices and methods described herein, the temperature changes resulting from delivery of PEF energy described herein were specifically evaluated. This evaluation was performed by monitoring the output from a thermocouple embedded within the distal electrode tip of the catheter electrode using a handheld digital multimeter (Klein Tools, MM700). Video recordings of the multimeter readout were used to trace the evolution of temperature change in the catheter electrode during delivery. A treatment dose designed to achieve 6.6mm of treatment depth was delivered in the left atrium with a cadence representing a patient heart rate of 119 bpm. The resulting thermal profile for the catheter electrode is provided in Fig. 24. Here it can be seen that even in a higher dose situation, the temperature never exceeds 45°C, well below the threshold for rapid onset of extracellular protein denaturation (65°C). The temperature is also noted to return to within 1°C of baseline by approximately 5s after reaching this peak temperature. Therefore, it is evident that thermal damage (extracellular protein denaturation) is not generated in the cardiac tissue, reducing the chances of adverse events and anatomical deficits such as pulmonary vein stenosis resulting from the treatment. This also eliminates the generation of surface char or thermal injury which impedes energy delivery to underlying tissue, reducing the ability to generate transmural lesions.

However, it may be appreciated that, in some embodiments, the system 100 includes temperature sensing and/or control measures for various purposes. In some embodiments, temperature is sensed and controlled to ensure that the temperature remains in the range of 30-65°C, 30-60°C, 30-55°C, 30-50°C, 30-45°C, 30-35°C. Thus, lesions are not created by thermal injury as the temperature of the tissue remains below a threshold for thermal ablation. In some embodiments, a temperature sensor is used to measure electrode and/or tissue temperature during treatment to ensure that energy deposited in the tissue does not result in any clinically significant tissue heating. For example, in some embodiments, a temperature sensor monitors the temperature of the tissue and/or electrode, and if a pre-defined threshold temperature is exceeded (e.g. 65°C), the generator alters the algorithm to automatically cease energy delivery or modifies the algorithm to reduce temperature to below the pre-set threshold. For example, in some embodiments, if the temperature exceeds 65°C, the generator reduces the pulse width or increases the time between pulses and/or packets (e.g. delivering energy every other heart beat, every third heart beat, etc.) in an effort to reduce the temperature. This can occur in a pre-defined step-wise approach, as a percentage of the parameter, or by other methods. It may be appreciated that temperature sensors may be positioned on electrodes (as illustrated in Fig. 25), adjacent to electrodes, or in any suitable location along the distal portion of the catheter 102. Alternatively or in addition, sensors may be positioned on one or more separate instruments.

In other embodiments, temperature is sensed to assess lesion formation. This may be particularly useful when generating lesions in anatomy having target tissue areas of differing thicknesses. A rapid rise in temperature indicates that the lesion has penetrated the depth of the tissue and is nearing completion. Sensing such changes in temperature may be particularly useful when generating lesions in thicker tissues or tissues of unknown depth.

In some embodiments, the treatment catheter 102 includes irrigation to assist in controlling the temperature of the delivery electrode 122 or surrounding tissue. In some instances, irrigation cools the delivery electrode 122, allowing more PEF delivery per time without increasing any potential heat-mediated damage. In some instances, irrigation also reduces or prevents coagulation near the tip of the catheter 102. It may be appreciated that irrigation may be activated, increased, reduced or halted based on information from one or more sensors, particularly one or more temperature sensors.

Such cooling is achieved by delivering fluid, such as isotonic saline solution, through a lumen in the catheter 102 that exits through one or more irrigation ports along the distal end of the catheter 102. The fluid may be chilled fluid, room temperature fluid or warmed fluid. The fluid flow can be driven by a variety of mechanisms including a gravity driven drip, a peristaltic pump, a centrifugal pump, etc. In some embodiments, the irrigation has a flow rate of 0.1-10ml/min, including 1 ml/min, 2 ml/min, 3 ml/min, 4 ml/min, 5 ml/min or more. In some embodiments, the flow rate is sensed by electrical or mechanical flow sensing mechanisms. In some embodiments, the temperature of the fluid is measured, and in other embodiments the temperature of the fluid is modified, such as warmed or cooled, as it is pumped into the treatment catheter 102, such as based on the measured temperature. In some embodiments, the fluid flow rate is determined based on the measured temperature of the tissue to be treated.

In some embodiments, the pump is in electrical communication with the generator 108 wherein the fluid flow rate is modified by the generator 108 based on the status of energy delivery to the treatment catheter 102. For example, in some embodiments, fluid flow rate is increased during energy delivery. Likewise, in some embodiments, fluid flow rate is increased a predetermined amount to time prior to energy delivery and/or at a predetermined time(s) during energy delivery. Alternatively or in addition, fluid flow may be controlled on demand by the user. It may be appreciated that the pump may communicate with the generator 108 to operate at different speeds based on various aspects of the energy delivery algorithm 152. In some embodiments, sensing of flowrate and communication with the generator 108 is used to prevent energy delivery if irrigation is not running. In other embodiments, selection of an energy delivery algorithm 152 in turn selects a fluid flow rate appropriate for the energy delivery algorithm 152.

In some embodiments, at least one irrigation port is located along an electrode and/or optionally at least one irrigation port is located along the shaft 120. In some embodiments, as illustrated in Fig. 25, the treatment catheter 102 comprises a delivery electrode 122 having the form of a cylindrical "solid tip". In some embodiments, one or more irrigation ports 822 are located along a distal face of the cylindrical electrode tip. This allows delivery of fluid directly out the distal tip of the catheter 102. In some embodiments, one or more irrigation ports 822' are located along a side surface of the cylindrical delivery electrode 122. In some embodiments, one or more irrigation ports 822" are located along an edge of the cylindrical delivery electrode 122, such as along the transition between the shaft 120 and the delivery electrode 122. This allows the fluid to flow through the shaft 102 and then flow over the exterior of the delivery electrode 122. It may be appreciated that irrigation ports 822 may be located at a plurality of locations, including proximally along the shaft 120.

In some embodiments, irrigation also mitigates the effects of macrobubble and microbubble formation. Gas embolization is a concern with many PEF therapies, particularly due to the possible production of small "microbubbles" at the delivery electrode 122. Studies have shown that as little as 0.1 mL of air in the coronary arteries is able to cause myocardial damage. It is believed that larger bubbles have a higher probability of embolization, potentially leading to ischemic events, since smaller bubbles more easily dissolve back into the bloodstream. Typically, microbubbles form on a surface of an electrode and increase in size as more energy is delivered. When the microbubbles are sufficiently large, the bubbles dislodge from the electrode and float away. Irrigation at the electrode creates a flow of solution that dislodges the bubbles when they are smaller and can, therefore, more easily dissolve before reaching the coronary arteries. Thus, irrigation ports 822 that allow the fluid to flow over the exterior of the delivery electrode 122 particularly assist reducing microbubble formation.

Interface Connector

Conventional electroanatomic mapping (EAM) systems are often used to provide real-time three-dimensional anatomic information to guide conventional catheter ablation without radiation exposure or the shortcomings of fluoroscopy. EAM systems typically use either magnetic- or impedance-based mapping algorithms, or a combination of both, to visualize and generate models and maps (e.g. CARTO® systems by Biosense Webster/Johnson&Johnson, EnSite™ systems by St. Jude Medical/Abbott). Using these systems, electrophysiologists create a real time 3D representation of cardiac anatomy and electrical activity by positioning a mapping catheter in various regions of the heart. When the doctor moves the catheter in a sweeping motion, the systems track the catheter's location. In a procedure, the table where the patient lies has a magnetic frame that generates a magnetic field that tracks movement of the catheter via magnetic sensors in the catheter. Additionally, patches on the patient's skin emit a current that allows the systems to track the impedance changes on the electrodes of the catheter. Another EAM system, the KODEX-EPD system (Philips) has been introduced which involves a newer approach to cardiac imaging that shows real-time HD imaging delivering true anatomy and creates voltage and activation maps.

Electroanatomic mapping systems are sometimes called multi-modality mapping or image integration systems because they can show pictures or data from other sources. For instance, patient computed tomography (CT) or magnetic resonance image (MRI) scans taken a few days or weeks before the procedure may be loaded onto at least EnSite™ or CARTO® and matched with the real time 3D models of the heart. This is achieved by identifying and matching unique cardiac structures between the 3D model and the CT/MRI scan using the system's image integration tool After several common areas on the two images are identified, the system merges/fuses the 3D model with the CT/MRI scan into one 3D model. It usually takes about 15 minutes to complete this process; however, the positioning of anatomy can even change within just a week, so if the pre-procedure scan does not easily correspond with the real time view of the heart, it can take much longer.

Electroanatomic mapping systems also provide real time data on electrical activity within the heart so that electrophysiologists can confirm that conduction block has been achieved. In some instances, the systems can provide other real time information, such as atrial pressure and volume, so as to monitor the patient during the procedure.

Thus, electroanatomic mapping systems integrate at least three important functionalities, namely (a) non-fluoroscopic localization of electrophysiological catheters in three-dimensional space; (b) analysis and 3D display of activation sequences computed from local or calculated electrograms and 3D display of electrogram voltage; and (c) integration of this 'electroanatomic' information with non-invasive images of the heart, such as computed tomography or magnetic resonance images.

In some embodiments, during an electrophysiology (EP) procedure in which pulsed field energy is utilized as the treatment energy, the electrodes of the treatment catheter are used for multiple purposes. For example, in some embodiments, in addition to delivery of PEF energy, the electrodes are used to measure low-voltage intracardiac electrograms and/or measure impedance for electroanatomic mapping systems. To do this, the electrodes of the cardiac treatment catheter are simultaneously connected to a pulsed electric field generator, as well as several pieces of EP equipment (e.g. EP recording system, electroanatomic mapping system such as CARTO®, EnSite™ or KODEX-EPD). When these systems share the same electrical conductor, the signals of the various pieces of equipment can interfere with each other.

An interface connector is provided that minimizes the interference between the various pieces of equipment connected to the contacts (e.g. electrodes, sensors, etc.) of the cardiac treatment catheter. The interface connector comprises a switching system such that EP signal amplifiers (e.g. EP recording systems and electroanatomic mapping systems such as CARTO®, Ensite™ or KODEX-EPD) are isolated when PEF energy is being delivered via the catheter. In some embodiments, such isolation is achieved using high-voltage relays. When PEF energy is not being delivered via the catheter, the PEF generator is similarly isolated from the EP signal amplifiers, such as with the use of high-voltage relays.

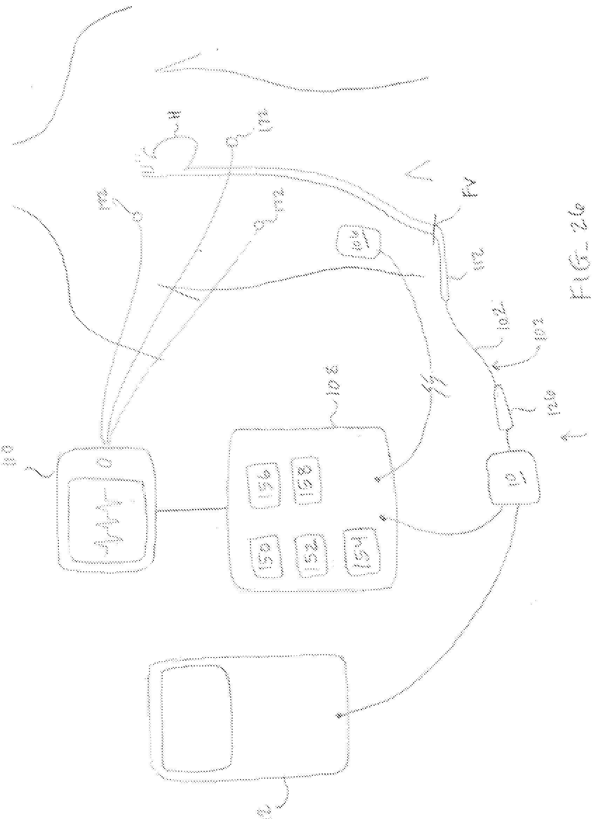

Fig. 26 illustrates an example setup wherein an interface connector 10 is utilized with an embodiment of a tissue modification system 100. In this embodiment, the tissue modification system includes a specialized catheter 102 (however, a conventional catheter 101 delivering PEF energy may alternatively be used), a high voltage biphasic waveform generator 108 and at least one distinct energy delivery algorithm 152. It may be appreciated that additional accessories and equipment may be utilized, such as an external cardiac monitor 110 connected to external electrodes 172 which are applied to the patient P to acquire the ECG. In this embodiment, the treatment catheter 102 is designed to be monopolar, wherein the distal end of the catheter 102 has as at least one delivery electrode and a return electrode 106 is positioned upon the skin outside the body, typically on the thigh, lower back or back. In this embodiment, the heart is accessed via the right femoral vein FV by a suitable access procedure, such as the Seldinger technique. Typically, an introducer sheath 112 is inserted into the femoral vein FV which acts as a conduit through which various catheters and/or tools may be advanced, including the treatment catheter 102. As illustrated in Fig. 26, the distal end of the catheter 102 is advanced through the inferior vena cava, through the right atrium, through a transseptal puncture, and into the left atrium so as to access the entrances to the pulmonary veins. In this embodiment, the catheter 102 is used to perform cardiac mapping which refers to the process of identifying the temporal and spatial distributions of myocardial electrical potentials during a particular heart rhythm. Cardiac mapping during an aberrant heart rhythm aims at elucidation of the mechanisms of the heart rhythm, description of the propagation of activation from its initiation to its completion within a region of interest, and identification of the site of origin or a critical site of conduction to serve as a target for treatment. Once the desired treatment locations are identified, the catheter 102 is utilized to deliver the treatment energy.

In some embodiments, the proximal end of the treatment catheter 102 is electrically connected with the interface connector 10. In the embodiment illustrated in Fig. 26, the interface connector 10 is electrically connected with the waveform generator 108 and a separate external device 12, such as one having the capability of providing the electroanatomic mapping procedure (e.g. a CARTO® system, Ensite™ system, KODEX-EPD system, etc.). In addition, as illustrated in this embodiment, the generator 108 is connected with an external cardiac monitor 110 to allow coordinated delivery of energy with the cardiac signal sensed from the patient P.

It may be appreciated that in some instances the interface connector 10 is connected directly to an electroanatomic mapping system, such as to a patient interface unit of the electroanatomic mapping system, with the use of a cable. However, it may also be appreciated that in other embodiments, the interface connector 10 is connected to a pin box, break out box, input-output box, junction box or other accessory that is then connected to the electroanatomic mapping system, such as with a specialized cable. The specialized cable spreads the multi-cable line into individual component connectors or tip pins that are insertable into receptacles in the pin box. This allows access to each electrode individually. The pin box is then connected to the electroanatomic mapping system. It may also be appreciated that the interface connector 10 may include features of the pin box so as to eliminate a separate pin box. Thus, the interface connector 10 may include receptacles for receiving tip pins and the associated electronics.

Figs. 27-28 illustrate an embodiment of an interface connector 10 having a switching system 13. As shown, the proximal end of the catheter 102 is electrically connected with a first port 20 along the interface connector 10. The catheter 102 includes at least one electrode (e.g. a delivery electrode 122 and optionally one or more additional electrodes 125) near its distal tip. The interface connector 10 includes a second port 22 for electrical connection to the separate external device 12 (such as to EP signal amplifiers of an electroanatomic mapping system) and a third port 24 for electrical connection to the generator 108. The switching system 13 comprises a path 30 and a branched path 32 of electrically conductive wires or traces. The first port 20 is connected to the second port 22 by the path 30 of electrically conductive wires or traces. A branched path 32 of electrically conductive wires or traces branches from path of electrical wiring 30 and connects to the third port 24. Each path 30, 32 includes at least one switch 36, 38 wherein opening of the switch(es) 36, 38 prevent passage of the signals therethrough and closing of the switch(es) 36, 38 allows passage therethrough. Thus, passage of the signals between the ports 20, 22, 24 can be controlled by selectively opening and closing the switches 36, 38. For example, Fig. 27 illustrates the switch(es) 36 within the path 30 being closed, thereby allowing input signals from the electrode(s) 122, 125 (e.g. EP mapping signals or intracardiac electrogram signals, etc.) to pass from the catheter 102 to the separate external device 12, while the switch(es) 38 within the path 32 is/are open so as to prevent passage of electrical signals from the generator 108 to the electrode(s) 122, 125. Fig. 28 illustrates the switch(es) 38 within the path 32 being closed, thereby allowing treatment energy from the generator 108 (connected to the port 24) to be delivered to the electrode(s) 122, 125, while the switch 36 within the path 30 is open so as to prevent passage of electrical signals to the separate external device 12 (connected to the port 22). In certain embodiments, each of the switches 36, 38 is implemented using a respective high voltage relay.

In Figs. 27-28, the catheter 102 is shown as having four electrodes 122, 125, but can alternatively have more or less than four electrodes. Here, each of the ports 20, 23, and 24 includes a separate electrical terminal corresponding to each of the electrodes 122, 125 with each terminal providing an electrical connection between one of the electrodes 122, 125 and one of the electrically conductive wires or traces within each of the paths 30, 32. Similarly, each of the paths 30, 32 includes a separate electrically conductive wire or trace, and a separate switch 36, 38, corresponding to each of the electrodes 122. 125. All of the switches 36 within the path 30 can be simultaneously opened and closed. Alternatively, one or more of the switches 36 within the path 30 can be opened while one or more other one(s) of the switches 36 is/are closed, where only certain one(s) of the electrodes 122, 125 are to be used for sensing an input signal. Similarly, all of the switches 38 within the path 33 can be simultaneously opened and closed. Alternatively, one or more of the switches 38 within the path 32 can be opened while one or more other one(s) of the switches 38 is/are closed, where only certain one(s) of the electrodes 122, 125 are to be used for delivering treatment energy.

When the second port 22 (e.g. electrically connect to EP signal amplifiers) is switched out during PEF energy delivery, as in Fig. 28, the electrophysiologist is not able to visualize the catheter 102 location and view intracardiac electrogram signals. Therefore, it is typically desired to switch-out the second port 22 (e.g. electrically connect to EP signal amplifiers) for the small amount of time in which a portion of energy is delivered (e.g. as a "packet").

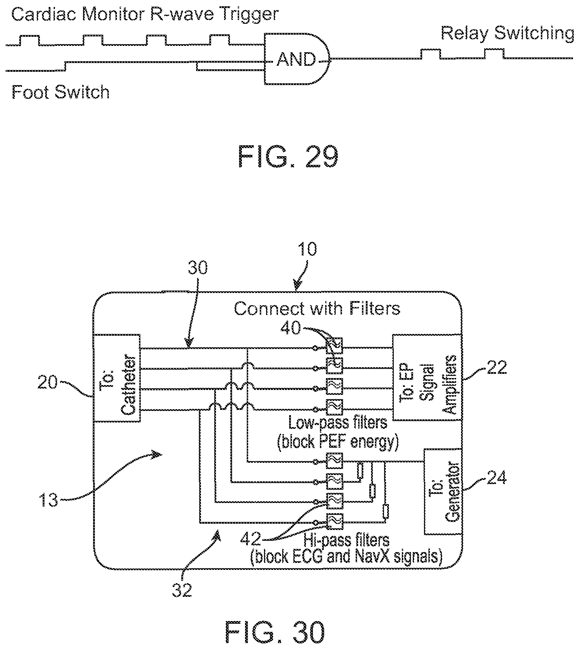

Treatment energy delivery may be actuated by a variety of mechanisms, such as with the use of a button on the catheter 102 or a foot switch operatively connected to the generator 108. Such actuation typically provides a single energy dose. The energy dose may be defined at least in part by the number of packets delivered and the voltage of the packets. The energy dose can also be defined by the number of pulses within each packet and the pulse width of each of the pulses within each packet but is not limited thereto. In some embodiments, such treatment energy delivery is synchronized with the heartbeat of the patient P, such as by synchronizing delivery of the packets with the use of an R-wave trigger from a cardiac monitor 110. In some embodiments, the switching of the paths 30, 32 or relays are controlled based on the "AND" logic of the generator footswitch signal and the R-wave trigger signal of the cardiac monitor, such as illustrated in Fig. 29. Only when both the foot switch and R-wave trigger signal are enabled are the high-voltage relays configured for delivering PEF energy and the EP signal amplifiers are isolated. In certain embodiments, the R-wave trigger signal is enabled at a programmable delay following each detection of an R-wave within a sensed intracardiac electrogram. In all other logic states of the foot switch and R-wave trigger, the high-voltage relays are configured for not delivering energy and the generator 108 is isolated from all mapping signals and intracardiac electrograms, by opening the switches 38 that are implemented as the relays.

In some embodiments, the switching system 13 utilizes signal filtering rather than switches to accomplish the intended function. This is typically dependent on the frequency ranges of the various signals. For example, Fig. 30 illustrates an embodiment of a connector 10 suitable for when the signals between the catheter 102 and the EP signal amplifiers are lower in frequency than the PEF output. In this embodiment, the signal lines to the EP signal amplifiers include low-pass filters 40 to exclude the high frequency PEF energy. Similarly, the signal lines between the catheter 102 and generator 108 include high-pass filters 42 to exclude all lower frequency signals using by electroanatomic mapping systems and EP recording system.

In some instances, it is desired that the catheter 102 is in communication with the generator 108, such as to deliver PEF energy, while in communication with the separate external device 12, such as to monitor contact force. If the catheter 102 is not configured for this situation, portions of the catheter 102 (e.g. one or more electrodes, internal wiring, etc.) may overheat and/or fail. This can be mitigated by a variety of design features. In some embodiments, the interface connector 10 is adapted to control the passage of signals in coordination with the PEF energy delivery. For example, in some embodiments, the catheter 102 is used for impedance sensing, ECG sensing, contact force sensing and magnetics, to name a few. In such embodiments, some features may be used simultaneously without detrimental effects. In some instances, impedance sensing and ECG sensing may be utilized with limited or no interference or negative effects with PEF delivery. Thus, in some embodiments, particular signals may be allowed to pass during delivery of the PEF energy. This may be achieved by manipulating the appropriate switches 36, 38. In this example, switches 36, 38 along paths of electrically conductive wires or traces associated with impedance sensing and ECG sensing are both open. In some embodiments, particular signals are more likely to cause interference or detrimental effects with PEF delivery, such as signals related to contact force sensing and magnetics. In such embodiments, these signals may be blocked during delivery of the PEF energy. This may also be achieved by manipulating the appropriate switches 36, 38. In this example, one or more of the switches 36 within the path 30 along electrically conductive wires or traces associated with contact force sensing and/or magnetics are closed during PEF energy delivery and open otherwise. Similarly, one or more of the switches 38 within the path 32 along these electrically conductive wires or traces are open during PEF energy delivery and closed otherwise. It may be appreciated that any combination of features may be allowed or blocked at any given time by either manipulation of the switches 36, 38 or by alternative design. It may also be appreciated that, due to the nature of PEF delivery, such blocking of access to the electroanatomic mapping system may be of such short duration that it may be unnoticeable to the user. For example, contact force sensing and/or imaging may appear continuous to the user simultaneous with these periods of blocking. It may be appreciated that in other embodiments one or more signals may be manipulated so that the catheter 102 may be in electrical communication with the generator 108 and the separate external device 12 at the same time.

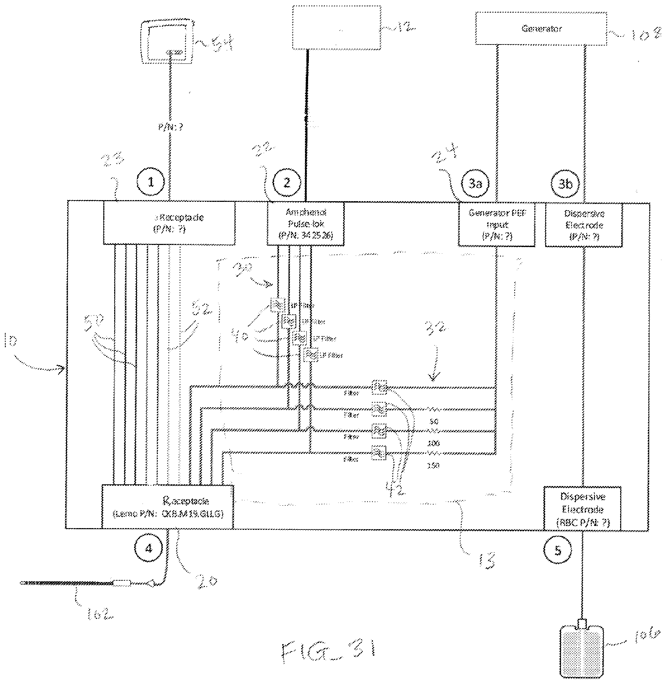

Alternatively, as illustrated in Fig. 31, signals unrelated to delivery of energy or cardiac mapping may travel along a separate pathway from paths 30, 32. For example, signals related to contact and/or contact force sensing may travel along paths of conductive wires or traces 50 that are separate from the paths 30, 32 involved with switching between the generator 108 and the external device 12. Here, the contact and/or contact force sensing traces 50 extend from port 20 to port 23 which in turn connects with a module 54 having components related to the measurement of contact and/or contact force. This separates the activity related to contact and/or contact force sensing from the delivery of energy, mapping, etc. Likewise, signals related the temperature sensing may travel along paths of conductive wires or traces 52 that are also separate from the paths 30, 32 involved with switching between the generator 108 and the external device 12. Here, the temperature sensing traces 52 extend from port 20 to port 23 which in turn connects with the module 54 (or optionally a separate module) having components related to the measurement of temperature. This separates the activity related to temperature sensing from the delivery of energy, mapping, etc.

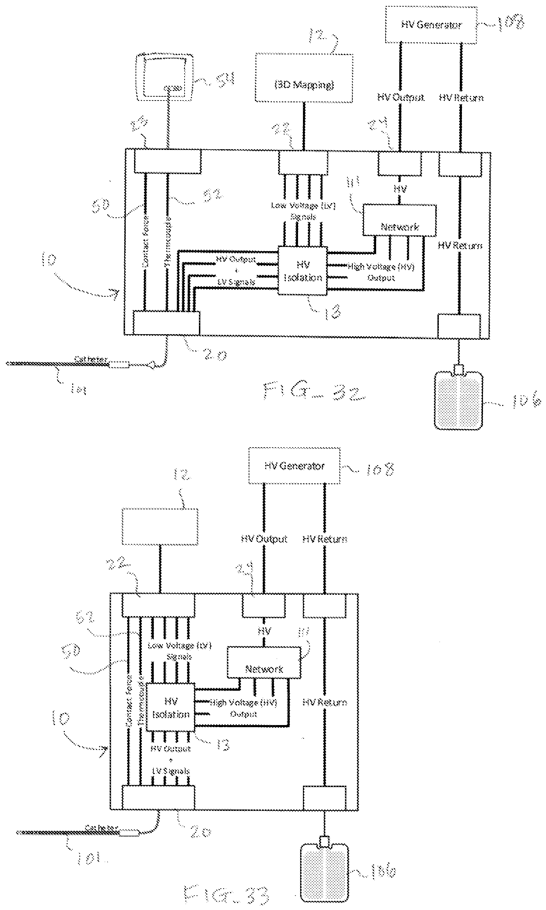

In some embodiments, particularly when utilizing a conventional ablation catheter 101, a component network 111 is included in an interface connector 10, such as illustrated in Figs. 32-33. Fig. 32 illustrates an embodiment of an interface connector 10 similar to Fig. 31 with the addition of a component network 111. Here, the component network 111 is disposed between the switching system 13 and the port 24 which connects to the generator 108. This set up may be used when connecting the catheter 102 to separate devices for mapping and sensing (e.g. contact force sensing, temperature sensing, etc.). Therefore, the switching system 13 is disposed between port 20 and port 22. This may be particularly the case when the external device 12 comprises an Ensite™ electroanatomic mapping system and the module 54 comprises a Tactisys™ System, which provides components for contact force and temperature monitoring. Similarly, Fig. 33 illustrates a component network 111 disposed between the switching system 13 and the port 24 which connects to the generator 108. However, this set up may be used when connecting the catheter 102 to a single device that provides mapping and sensing (e.g. contact force sensing, temperature sensing, etc.). Again, the switching system 13 is disposed between port 20 and port 22, however, port 23 has been eliminated so that the contact and/or contact force sensing traces 50 and the temperature sensing traces 52 extend from port 20 to port 22. This may be particularly the case when the external device 12 comprises a CARTO® electroanatomic mapping system which includes components for contact force and temperature monitoring.

Fig. 34 illustrates an embodiment of a tissue modification system 100 for use with a patient (not shown) comprising a treatment catheter (either a specialized catheter 102 or a conventional ablation catheter 101), a return electrode 106, a foot switch 168, an interface connector 10, a waveform generator 108, a separate external device 12 (e.g. having the capability of providing the electroanatomic mapping procedure), an external cardiac monitor 110, and various other accessories including a pin box 171. Fig. 35 illustrates an embodiment of a tissue modification system 100 for use with a patient P wherein the treatment catheter comprises a particular conventional RF catheter 101, a Tacticath™ ablation catheter. Again, the system 100 includes a return electrode 106, a foot switch 168, an interface connector 10, a waveform generator 108, a separate external device 12 (e.g. having the capability of providing the electroanatomic mapping procedure), an external cardiac monitor 110, and various other accessories including a pin box 171. In addition, the system 100 includes a module 54 comprising a Tactisys™ System for contact force and temperature acquisition.

The system 100 produces treatment effects that are readily apparent in real-time while monitoring treatment delivery and progression. In some instances, a strong attenuation of the ECG signal is apparent at the treatment catheter following treatment delivery. In addition, in some embodiments, voltage mapping is performed prior-to and following a single-site treatment, where changes in the voltage map are clearly evident confirming operation with 3D mapping systems and the ability to use them to track delivery progress as the treatment sites are connected to generate a continuous lesion of electrical conduction block.

Alternative Treatment Catheter Designs

The systems and devices described herein may alternatively be used with a variety of other types and styles of treatment catheters 102. In some embodiments, the treatment catheters 102 are designed to deliver focal therapy and in other embodiments, the treatment catheters 102 are designed to deliver "one shot" therapy. Focal therapy is considered to be a therapy wherein the energy is delivered in a sequence, such as the repeated application of energy in point by point fashion around a pulmonary vein to create a circular treatment zone, such as previously illustrated in Fig. 4. One shot therapy is considered to be a therapy wherein the energy is delivered via the delivery electrode to the entire circumference of the entrance to the pulmonary vein in "one shot", however such delivery may be repeated if desired. This may optionally include rotation of the electrode 122 between "shots" if desired.

*Focal therapy*

As mentioned previously, focal therapy is typically performed with the use of a delivery electrode 122 having a cylindrical shape with a distal face, such as illustrated in Figs. 2A-2B. In some embodiments, the distal face of the delivery electrode 122 has a diameter of 3mm. In such embodiments, when the treatment catheter 102 is positioned perpendicularly to the tissue and the distal face is positioned against the tissue, the surface contact area is approximately $7mm^2$. When delivering PEF energy as described herein, such as with the following parameter values: 3300V/400kHz/40 cycles/30 packets, 3300V/400kHz/30 cycles/10 packets, 2000V/400kHz/40 cycles/30 packets, each packet of energy delivers 15 joules of energy. In such instances, the delivery electrode has a current density of approximately $2mm^2$.

It may be appreciated that focal therapy may be delivered with the use of alternative catheter designs and methods. For example, in some embodiments, the treatment catheter 102 is configured to provide focal therapy such as according to international patent application number PCT/US2018/067504 titled "OPTIMIZATION OF ENERGY DELIVERY FOR VARIOUS APPLICATIONS" which claims priority to Provisional Patent Application No. 62/610,430 filed December 26, 2017 and U.S. Provisional Patent Application No. 62/693,622 filed July 3, 2018, all of which are incorporated herein by reference for all purposes.

*One Shot Therapy*

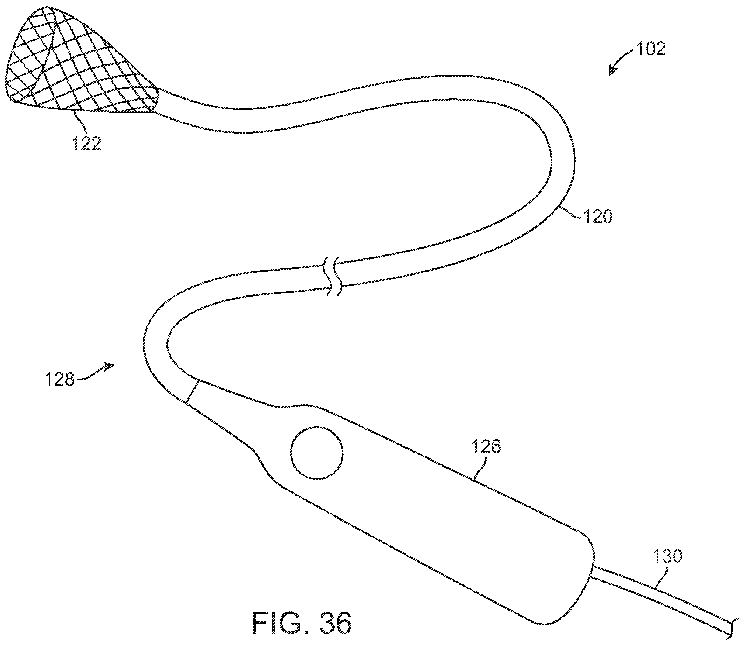

Fig. 36 illustrates an embodiment of a treatment catheter 102 configured to deliver "one-shot" therapy. One shot therapy is considered to be a therapy wherein the energy is delivered via the delivery electrode to the entire treatment area, such as the circumference of the entrance to the pulmonary vein, in "one shot", however such delivery may be repeated if desired. This may optionally include rotation of the electrode 122 between "shots" if desired.

In this embodiment, the catheter 102 comprises an elongate shaft 120 having a delivery electrode 122 near its distal end 124 and a handle 126 near its proximal end 128. Here, the delivery electrode 122 is configured to deliver energy to a larger area, such as an entire treatment area, particularly so as to create a continuous treatment area around a pulmonary vein to block conduction. In this embodiment, the delivery electrode 622 has a cup or funnel shape facing distally. The footprint created by the delivery electrode 622 has a diameter that is larger than the footprint created by a focal therapy catheter. This is to achieve a particular treatment in a single application. Thus, in some embodiments therapy is provided in one application of the electrode 122 to the tissue; however, it may be appreciated that in some instances the energy can be applied more than once if desired. The handle 126 is used to manipulate the catheter 102, particularly to steer the distal end 124 during delivery and treatment. Energy is provided to the catheter 102, and therefore to the delivery electrode 122, via a cable 130 that is connectable to the generator 108.

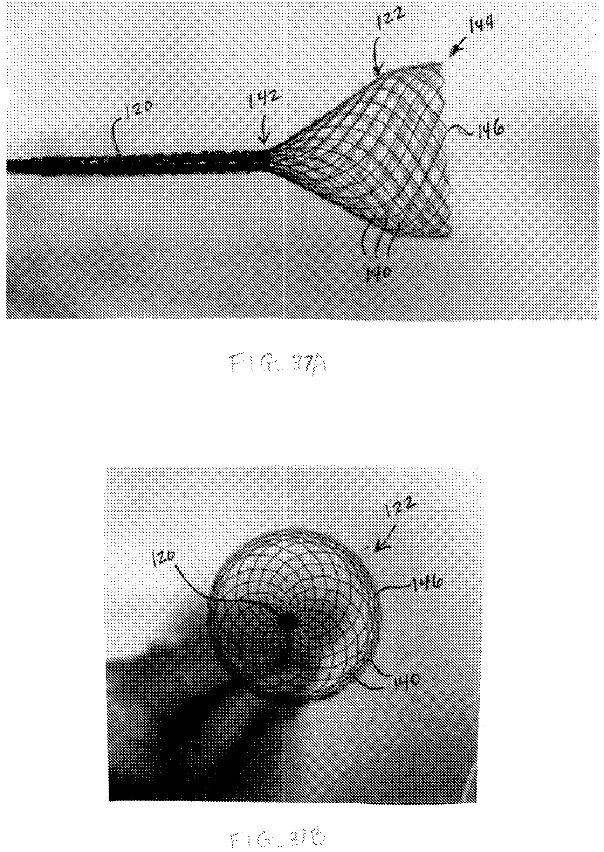

Figs. 37A-37B illustrate a similar embodiment of a delivery electrode 122 configured to deliver "one shot" therapy, wherein the delivery electrode 122 has a cup or funnel shape. In this embodiment, the electrode 122 comprises a plurality of wires 140 forming an expandable half-basket, wherein at least one of the wires acts as a delivery electrode. In this embodiment, the half-basket is attached to the shaft 120 near its proximal end 142 and has a circular open shape at its distal end 144 (i.e. free end). Thus, in the expanded configuration, the half-basket shape has a rim 146 along its distal end 144 forming a circular shape. In this embodiment, the wires 140 are curved along the rim 146 so as to create an atraumatic surface. Fig. 37B provides an end view of the embodiment of Fig. 37A. As illustrated, the rim 146 has a circular shape and the remainder of the half-basket has a woven appearance as it funnels down to the shaft 120. It may be appreciated that the plurality of wires 140 may be woven or intertwined in a variety of configurations. It may also be appreciated that the delivery electrode 122 may be self-expanding, wherein the electrode 122 resides in a collapsed configuration while maintained within a sheath and self-expands to the expanded configuration upon removal of the sheath. It may also be appreciated that the delivery electrode 122 may alternatively be expanded by other mechanisms, such as by inflation of a delivery balloon, etc.

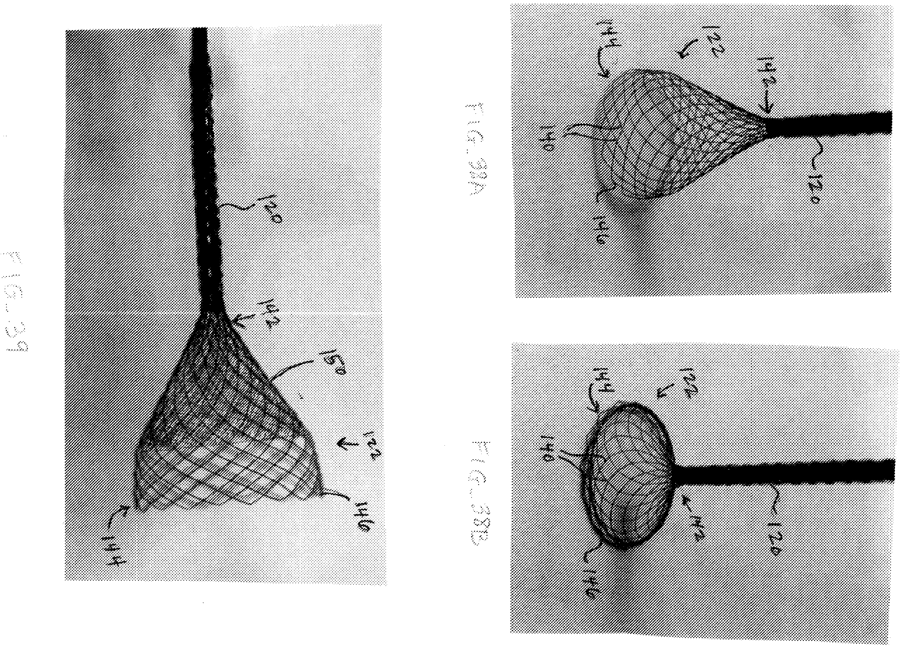

Figs. 38A-38B illustrate the application of the delivery electrode 122 of Figs. 37A-37B to a surface, such as an area of cardiac tissue. In these illustrations, the electrode 622 is shown contacting a benchtop to illustrate its shape changes during increasing application of contact force. It may be appreciated that similar shape changes will occur when contacting tissue, particularly cardiac tissue surrounding the pulmonary veins. One may visualize the circular opening of a pulmonary vein to align within the circular shape of the rim 146 so that the delivery electrode 122 contacts tissue surrounding the opening of the pulmonary vein. Fig. 38A illustrates the electrode 122 positioned against a surface so that the rim 146 is in contact with the surface. Here, the electrode 122 substantially maintains its free state having a funnel shape. Fig. 38B illustrates the electrode 122 with increased pressure against the surface. This causes a portion of the wires 140 to collapse together forming a larger rim 146. Thus, energy delivered by the plurality of wires 140 is greater along the rim 146 during application of pressure or contact force, due at least to the increase in wires 140 along the rim 146, compared to when minimal pressure or contact force is applied. It may be appreciated that Fig. 38B illustrates a condition of nearly maximum pressure or contact force applied, wherein the half-basket has nearly entirely collapsed creating a short funnel shape. It may be appreciated that a variety of different levels of pressure or contact force may be applied with varying configurations of the half-basket shape between these two configurations (i.e. Fig. 38A and Fig. 38B).

In some embodiments, at least a portion of one of the plurality of wires is insulated from a nearby wire of the plurality of wires. In some embodiments, the at least a portion of one of the plurality of wires is insulated leaving an exposed portion of wire so as to create an active area which concentrates the energy at a particular location along the target tissue. In some embodiments, the plurality of wires is simultaneously energizable. In other embodiments, at least some of the plurality of wires are individually energizable. In some embodiments, the delivery electrode 122 includes insulation covering at least a portion of the plurality of wires 140. For example, Fig. 39 illustrates an embodiment of a delivery electrode 122 as in Figs. 38A-38B wherein a portion of the plurality of wires 140 is covered by insulation 150. In this embodiment, the insulation 150 substantially covers the portion of the half-basket shape that does not collapse or minimally collapses into the rim 146 with the application of force against the tissue. Thus, the insulation 150 covers a portion of the proximal end 142 of the delivery electrode 122. This allows more energy to be delivered toward the distal end 144 of the delivery electrode 122, particularly the rim 146, since energy is not dissipated to the environment via the proximal end 142.

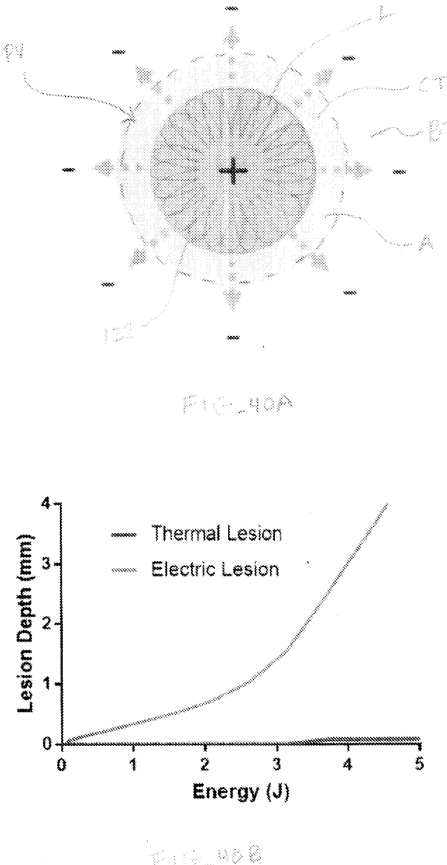

Fig. 40A provides a schematic illustration of a cross-section of a lumen L of a pulmonary vein PV surrounded by cardiac tissue CT and then body tissue BT therearound. In this illustration, the lumen L has a diameter of 25 mm and the cardiac tissue has a thickness of 4mm. A treatment catheter 102 is shown having a delivery electrode 122 at its distal end; the electrode 122 is illustrated as contacting the cardiac tissue CT in various locations simultaneously via the lumen L. It may be appreciated that in this example the delivery electrode 122 has a full-basket shape and is inserted into the lumen L. However, one may visualize that in other embodiments the half-basket shape may be inserted into the lumen L or the rim 146 may be positioned against the tissue surrounding the lumen L.

In this embodiment, the catheter 102 delivers the energy in a monopolar fashion wherein the energy flows from the delivery electrode 122 outwardly toward the surface of the body tissue BT (e.g. skin) and the return electrode (not shown) positioned thereon. This electric field creates a treatment area A of varying depth depending on the energy delivery algorithm 152. In this example, a treatment area A penetrating the thickness of 4mm is achieved. It may be appreciated that typically as the energy is increased, the size of the treatment area A likewise increases. An example of the association of energy and treatment area depth is illustrated in the graph of Fig. 40B (sloping line). Fig. 40B also illustrates the association between energy and thermal effects which is a flat line across the x-axis. Thus, the energy delivered is non-thermal.

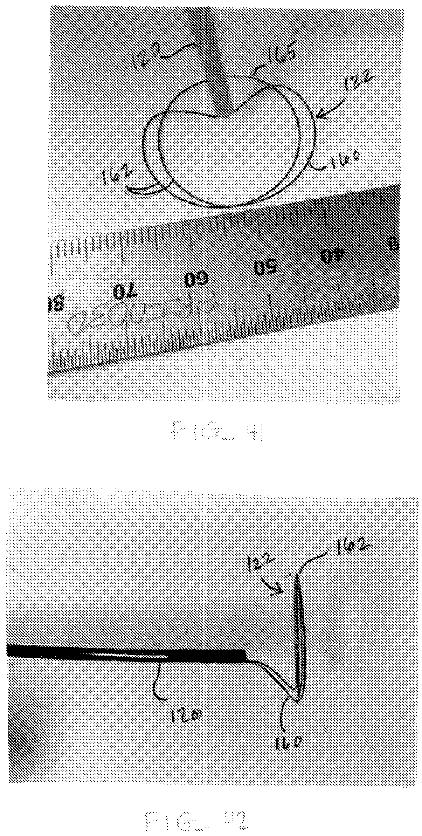

Another type of delivery electrode 122 configured to deliver "one shot" therapy has a looped shape. Typically, the looped shape is comprised of one or more loops arranged to form a continuous circular rim. Fig. 41 illustrates an embodiment of a delivery electrode 122 comprising an initial single loop. In this embodiment, the electrode 122 is formed from a shape-memory wire 160 that is able to twist and fold back upon itself during delivery to create a substantially circular rim 162. Thus, due to the folding action, the initial single loop forms a secondary loop 165 that extends around the rim 162 so that the circular rim 162 is substantially comprised of two layers of wire 160. In this embodiment, the secondary loop 165 extends around nearly the full perimeter of the rim 162 (i.e. together the initial loop and the secondary loop 165 form the rim 162); therefore, more than half of the perimeter of the rim 162 is comprised of two layers of wire. The circular rim 162 is typically substantially perpendicular to the shaft 120 when deployed, as illustrated in Fig. 42 which provides a side view of the delivery electrode 122 depicted in Fig. 41. This allows the rim 162 to be positioned against the cardiac tissue surrounding an entrance to a pulmonary vein. Thus, energy is delivered via the delivery electrode 122 to the entire circumference of the entrance to the pulmonary vein in "one shot", however such delivery may be repeated if desired. This may optionally include rotation of the electrode 122 between "shots" if desired. In this embodiment, the shaft 120 is offset and not concentric with the circular rim 162, however it may be appreciated in other embodiments the shaft 120 is concentric with the rim 162.

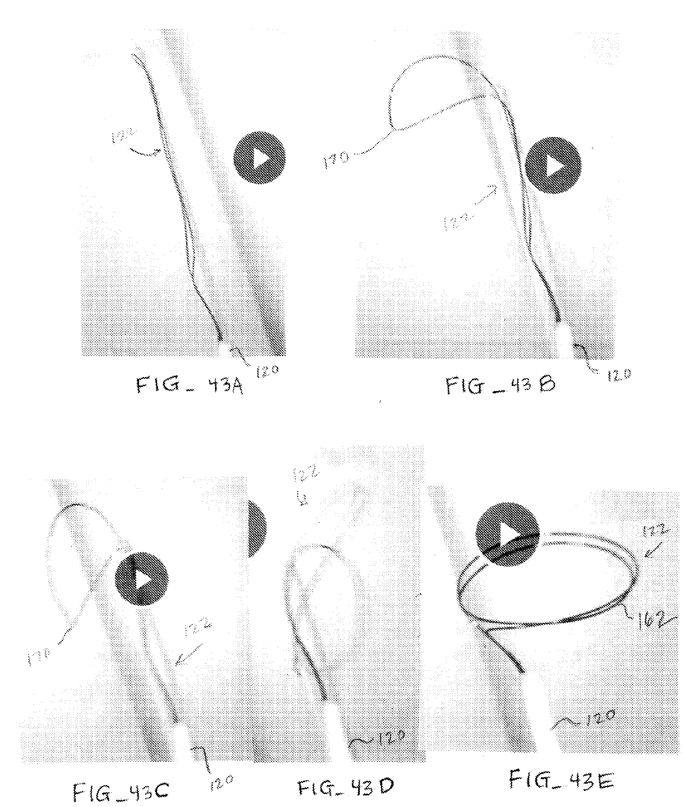

Figs. 43A-43E illustrate deployment of the delivery electrode 122 of Figs. 41-42. In this embodiment, the delivery electrode 122 is configured to be housed within the shaft 120, however, it may be appreciated that, alternatively, a sheath may be advanced over the catheter 102 to capture the electrode 122. To begin, the electrode 122 is unfolded and flattened into a relatively straightened configuration when housed within the shaft 120. This allows the electrode 122 to be collapsed into a small configuration, so as to allow for a small outer diameter shaft 120 for advancement through the vasculature. In this embodiment, the electrode 122 is deployed by advancement of the electrode 122 from the shaft 120. Fig. 43A illustrates the electrode 122 advanced from the distal end of the shaft 120 and having the initially straightened configuration. This is the initial loop. It may be appreciated that the deployment steps illustrated in Figs. 43A-43E are stills from a video wherein each step occurs in quick succession on its own. Thus, the electrode 122 self-configures into the deployed configuration upon release. Fig. 43B illustrates electrode 122 beginning to fold downward upon itself, revealing its looped shape. In this embodiment, the distal-most portion 170 of the loop curves and curls backwards in a proximal direction toward the shaft 120. Fig. 43C illustrates the distal-most portion 170 bending further downward so that the loop forms a V-shape. This V-shape is the beginning stages of the formation of the two layers of wire that create the rim 162. Fig. 43D illustrates the conversion of the electrode 122 from the V-shape into a double layered loop. This step occurs so quickly one cannot visualize it with the naked eye. In this embodiment, portions of the V-shape cross one another in a twisting and folding manner so as to form a double loop from the single loop. Fig. 43E illustrates the final configuration of the electrode 122 in its fully deployed state. Here, the loop of wire 160 has formed the circular rim 162 and is substantially comprised of two layers of the wire 160. As shown, the circular rim 162 is typically substantially perpendicular to the shaft 120 when deployed.

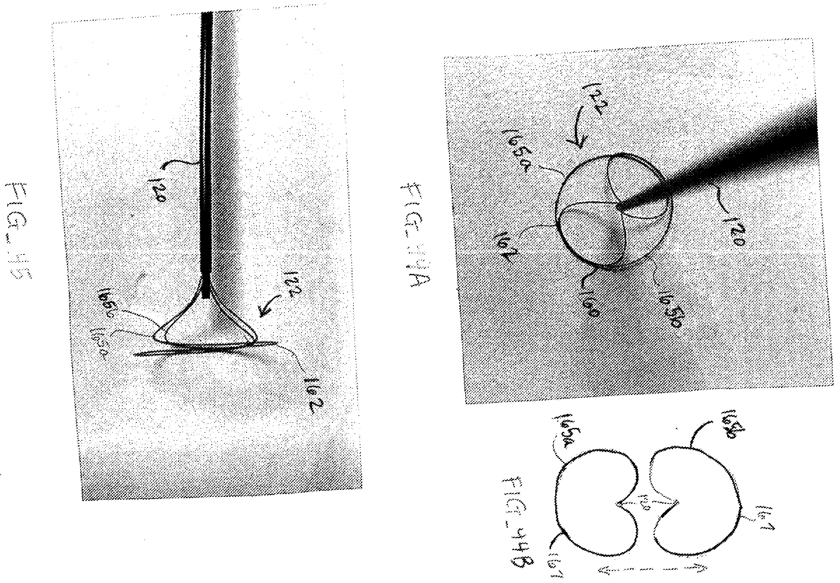

Figs. 44A-44B illustrate another embodiment of a delivery electrode 122 comprising loops of shape-memory wire 160 that create a substantially circular rim 162. As illustrated in Fig. 44A, in this embodiment, two loops 165a, 165b overlap so that together they form the circular rim 162. The two loops 165a, 165b have been isolated for visualization in Fig 44B. In this embodiment, each of the two loops 165a, 165b form an arc 167 traversing nearly 270 degrees of a circle around the shaft 120. Thus, the circular rim 162 is comprised of two layers of wire 160 in two portions where the loops 165a, 165b overlap. In this embodiment, the overlapping regions extend around approximately half of the perimeter of the rim 162 (each loop 165a, 165b extending around a quarter of opposite portions of the perimeter of the rim 162). Therefore, approximately half of the perimeter of the rim 162 is comprised of two layers of wire. However, it may be appreciated that the loops 165a, 165b may be configured to cover differing portions of the perimeter of the rim 162, including nearly the full rim. The circular rim 162 is typically substantially perpendicular to the shaft 120 when deployed, as illustrated in Fig. 45 which provides a side view of the delivery electrode 122 depicted in Fig. 44A. This allows the rim 162 to be positioned against the cardiac tissue surrounding an entrance to a pulmonary vein. Thus, energy is delivered via the delivery electrode 122 to the entire circumference of the entrance to the pulmonary vein in "one shot", however such delivery may be repeated if desired. This may optionally include rotation of the electrode 122 between "shots" if desired. In this embodiment, the shaft 120 is offset and not concentric with the circular rim 162, however it may be appreciated in other embodiments the shaft 120 is concentric with the rim 162.

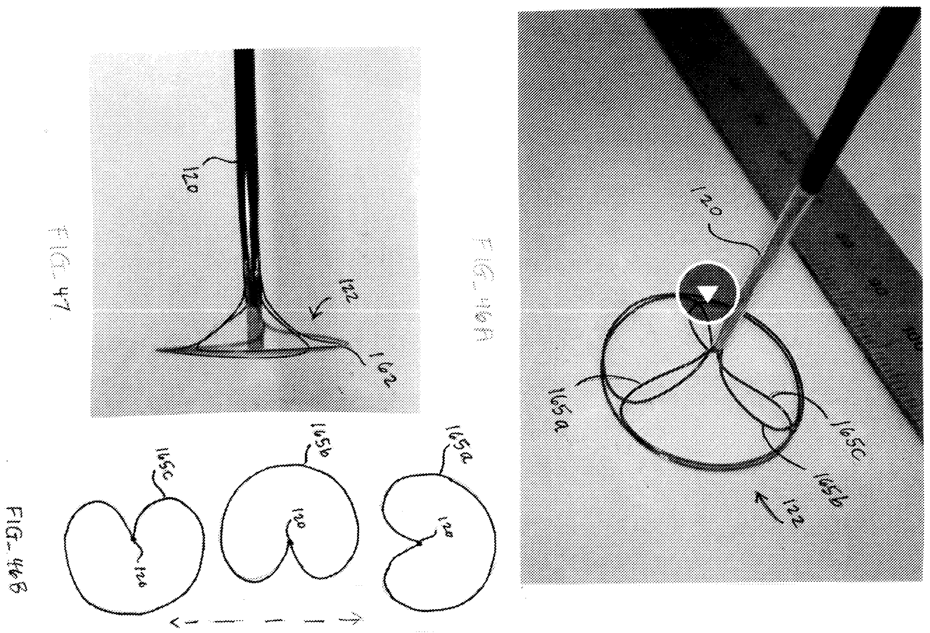

Fig. 46A illustrates another embodiment of a delivery electrode 122 comprising loops of shape-memory wire 160 that come together creating a substantially circular rim 162. In this embodiment, the electrode 122 is comprised of three loops 165a, 165b, 165c that extend at least partially around the rim 162 so that the circular rim 162 is comprised of two layers of wire 160 in three portions. The three loops 165a, 165b, 165c have been isolated for visualization in Fig 46B. In this embodiment, each of the three loops 165a, 165b, 165c form an arc 167 traversing nearly 270 degrees of a circle around the shaft 120. Thus, the circular rim 162 is comprised of three layers of wire 160 in three portions where the loops 165a, 165b, 165c overlap. In this embodiment, the overlapping regions extend around nearly the entire perimeter of the rim 162. In this embodiment (each loop 165a, 165b, 165c extending around approximately 1/3 of the perimeter of the rim 162). Therefore, approximately the full perimeter of the rim 162 is comprised of two layers of wire. The circular rim 162 is typically substantially perpendicular to the shaft 120 when deployed, as illustrated in Fig. 47 which provides a side view of the delivery electrode 122 depicted in Fig. 46A. This allows the rim 162 to be positioned against the cardiac tissue surrounding an entrance to a pulmonary vein. Thus, energy is delivered via the delivery electrode 122 to the entire circumference of the entrance to the pulmonary vein in "one shot", however such delivery may be repeated if desired. This may optionally include rotation of the electrode 122 between "shots" if desired. In this embodiment, the shaft 120 is offset and not concentric with the circular rim 162, however it may be appreciated in other embodiments the shaft 120 is concentric with the rim 162.

The above detailed description includes references to the accompanying drawings, which form a part of the detailed description. The drawings show, by way of illustration, specific embodiments in which the invention can be practiced. These embodiments are also referred to herein as "examples." Such examples can include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

In the event of inconsistent usages between this document and any documents so incorporated by reference, the usage in this document controls.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

The above description is intended to be illustrative, and not restrictive. For example, the above-described examples (or one or more aspects thereof) may be used in combination with each other. Other embodiments can be used, such as by one of ordinary skill in the art upon reviewing the above description. The Abstract is provided to comply with 37 C.F.R. §1.72(b), to allow the reader to quickly ascertain the nature of the technical disclosure. It is submitted with the understanding that it will not be used to interpret or limit the scope or meaning of the claims. Also, in the above Detailed Description, various features may be grouped together to streamline the disclosure. This should not be interpreted as intending that an unclaimed disclosed feature is essential to any claim. Rather, inventive subject matter may lie in less than all features of a particular disclosed embodiment. Thus, the following claims are hereby incorporated into the Detailed Description as examples or embodiments, with each claim standing on its own as a separate embodiment, and it is contemplated that such embodiments can be combined with each other in various combinations or permutations. The scope of the invention should be determined with reference to the appended claims.

## Claims

1. A system for creating a lesion in an area of cardiac tissue of a patient comprising:

   a treatment catheter including a catheter body including a distal portion and a proximal portion, the distal portion of the catheter body including conductive wires leading to a plurality of electrodes that are electrically isolated from one another;
   a generator electrically coupled to the treatment catheter and to the plurality of electrodes of the catheter, wherein the generator includes at least one energy delivery algorithm configured to provide an electric signal of pulsed electric field energy deliverable through the catheter to a selected one or more of the electrodes so as to create the lesion in the cardiac tissue; and
   a component network electrically coupled between the catheter and the generator, the component network configured to keep a potential difference between the one or more of the electrodes of the catheter that is selected for delivering pulsed electric field energy, and one or more other electrodes of the catheter that is not selected for delivering pulsed electric field energy, below a threshold potential difference which prevents arcing or shorting between one or more pairs of the electrically conductive wires or pairs of the plurality of electrodes.

2. A system as in claim 1, wherein the pulsed electric field energy comprises pulsed electric field energy having biphasic pulses in a plurality of packets.

3. A system as in acclaim 1 or 2, wherein the pulsed electric field energy has a voltage of at least 2000V.

4. A system as in claim 1, wherein the catheter is configured to avoid arcing between one or more pairs of the electrically conductive wires when receiving pulsed electric field energy having a voltage of up to a predetermined voltage level and wherein the pulsed electric field energy that is delivered by the signal generator comprises energy over the predetermined voltage level wherein the component network prevents arcing between one or more pairs of the electrically conductive wires or pairs of the plurality of electrodes.

5. A system as in claim 4, wherein the predetermined voltage level comprises 1000-1500 volts.

6. A system as in any of claims 1-5, wherein the catheter comprises a radiofrequency ablation catheter.

7. A system as in claim 1, wherein the component network comprises at least one resistor, inductor or diode.

8. A system as in claim 1, wherein the component network comprises a plurality of resistors.

9. A system as in claim 8, wherein each of the plurality of resistors is disposed between an electrically conductive wire electrically coupled to an electrode that is selected for delivering pulsed electric field energy and an electrically conductive wire electrically coupled to an electrode that is not selected for delivering pulsed electric field energy.

10. A system as in claim 8, wherein the plurality of electrodes comprises one electrode selected for delivering the pulsed electric field energy and three electrodes not selected for delivering the pulsed electric field energy, and wherein the plurality of resistors comprises

a first resistor disposed between a delivery electrically conductive wire electrically coupled to the electrode selected for delivering the pulsed electric field energy and a first electrically conductive wire electrically coupled to a first of the three electrodes not selected for delivering the pulsed electric field energy,

a second resistor disposed between the delivery electrically conductive wire electrically coupled to the electrode selected for delivering the pulsed electric field energy and a second electrically conductive wire electrically coupled to a second of the three electrodes not selected for delivering the pulsed electric field energy, and

a third resistor disposed between the delivery electrically conductive wire electrically coupled to the electrode selected for delivering the pulsed electric field energy and a third electrically conductive wire electrically coupled to a third of the three electrodes not selected for delivering the pulsed electric field energy.

11. A system as in claim 10, wherein a total resistance network combination of the first resistor, second resistor and third resistor have a maximum of 1000 to 1200 ohms.

12. A system as in claim 1, further comprising an external return electrode electrically coupleable with the signal generator and wherein the catheter delivers the pulsed electric field energy in a monopolar fashion with the external return electrode.

13. A system as in claim 1, wherein the treatment catheter has at least one contact, the system further comprising an interface connector that electrically couples the at least one contact to both the generator and an electroanatomic mapping system, wherein the interface connector is configured such that the electrode selected for delivering pulsed electric field energy is not electrically connected to both the generator and the electroanatomic mapping system simultaneously.

14. A system as claim 13, wherein the interface connector includes a switching system comprising a first path of at least one conductive wire between the electrode selected for delivering pulsed electric field energy and the generator and a second path of at least one conductive wire between the electrode selected for delivering pulsed electric field energy and the electroanatomic mapping system, wherein the switching system toggles the energy transmission between the first path and the second path.

**Patentansprüche**

1. System zum Erzeugen einer Läsion in einem Bereich des Herzgewebes eines Patienten, umfassend:

einen Behandlungskatheter, der einen Katheterkörper beinhaltet, der einen distalen Abschnitt und einen proximalen Abschnitt beinhaltet, wobei der distale Abschnitt des Katheterkörpers leitende Drähte beinhaltet, die zu einer Vielzahl von Elektroden führen, die elektrisch voneinander isoliert sind;

einen Generator, der elektrisch an den Behandlungskatheter und an die Vielzahl von Elektroden des Katheters gekoppelt ist, wobei der Generator mindestens einen Energieabgabealgorithmus beinhaltet, der dazu konfiguriert ist, ein elektrisches Signal gepulster elektrischer Feldenergie bereitzustellen, das durch den Katheter an eine ausgewählte eine oder mehrere der Elektroden abgegeben werden kann, um die Läsion im Herzgewebe zu erzeugen; und

ein Komponentennetzwerk, das elektrisch zwischen dem Katheter und dem Generator gekoppelt ist, wobei das Komponentennetzwerk dazu konfiguriert ist, eine Potentialdifferenz zwischen der einen oder den mehreren der Elektroden des Katheters, die zum Abgeben der gepulsten elektrischen Feldenergie ausgewählt ist, und einer oder mehreren anderen Elektroden des Katheters, die nicht zum Abgeben der gepulsten elektrischen Feldenergie ausgewählt ist, unter einer Schwellenpotentialdifferenz zu halten, die einen Lichtbogen oder Kurzschluss zwischen einem oder mehreren Paaren der elektrisch leitenden Drähte oder Paare der Vielzahl von Elektroden verhindert.

2. System nach Anspruch 1, wobei die gepulste elektrische Feldenergie gepulste elektrische Feldenergie mit biphasischen Pulsen in einer Vielzahl von Paketen umfasst.

3. System nach Anspruch 1 oder 2, wobei die gepulste elektrische Feldenergie eine Spannung von mindestens 2000 V aufweist.

4. System nach Anspruch 1, wobei der Katheter dazu konfiguriert ist, Lichtbögen zwischen einem oder mehreren Paaren der elektrisch leitenden Drähte zu vermeiden, wenn gepulste elektrische Feldenergie mit einer Spannung von bis zu einem vorbestimmten Spannungspegel empfangen wird, und wobei die gepulste elektrische Feldenergie, die von dem Signalgenerator abgegeben wird, Energie über dem vorbestimmten Spannungspegel umfasst, wobei das Komponentennetzwerk Lichtbögen zwischen einem oder mehreren Paaren der elektrisch leitenden Drähte oder Paaren der Vielzahl von Elektroden verhindert.

5. System nach Anspruch 4, wobei der vorbestimmte Spannungspegel 1000-1500 Volt umfasst.

6. System nach einem der Ansprüche 1-5, wobei der Katheter einen Radiofrequenzablationskatheter umfasst.

7. System nach Anspruch 1, wobei das Komponentennetzwerk mindestens einen Widerstand, einen Induktor oder eine Diode umfasst.

8. System nach Anspruch 1, wobei das Komponentennetzwerk eine Vielzahl von Widerständen umfasst.

9. System nach Anspruch 8, wobei jeder der Vielzahl von Widerständen zwischen einem elektrisch leitenden Draht, der elektrisch an eine Elektrode gekoppelt ist, die zum Abgeben der gepulsten elektrischen Feldenergie ausgewählt ist und einem elektrisch leitenden Draht, der elektrisch an eine Elektrode gekoppelt ist, die nicht zum Abgeben der gepulsten elektrischen Feldenergie ausgewählt ist, angeordnet ist.

10. System nach Anspruch 8, wobei die Vielzahl von Elektroden eine Elektrode umfasst, die zum Abgeben der gepulsten elektrischen Feldenergie ausgewählt ist, und drei Elektroden, die nicht zum Abgeben der gepulsten elektrischen Feldenergie ausgewählt sind, und wobei die Vielzahl von Widerständen umfasst

einen ersten Widerstand, der zwischen einem elektrisch leitenden Abgabedraht, der elektrisch an die Elektrode gekoppelt ist, die zum Abgeben der gepulsten elektrischen Feldenergie ausgewählt ist, und einem ersten elektrisch leitenden Draht, der elektrisch an eine erste der drei Elektroden gekoppelt ist, die nicht zum Abgeben der gepulsten elektrischen Feldenergie ausgewählt ist, angeordnet ist,
einen zweiten Widerstand, der zwischen dem elektrisch leitenden Abgabedraht, der elektrisch an die Elektrode gekoppelt ist, die zum Abgeben der gepulsten elektrischen Feldenergie ausgewählt ist und einem zweiten elektrisch leitenden Draht, der elektrisch an eine zweite der drei Elektroden gekoppelt ist, die nicht zum Abgeben der gepulsten elektrischen Feldenergie ausgewählt ist, angeordnet ist, und
einen dritten Widerstand, der zwischen dem elektrisch leitenden Draht, der elektrisch an die Elektrode gekoppelt ist, die zum Abgeben der gepulsten elektrischen Feldenergie ausgewählt ist, und einem dritten elektrisch leitenden Draht, der elektrisch an eine dritte der drei Elektroden gekoppelt ist, die nicht zum Abgeben der gepulsten elektrischen Feldenergie ausgewählt ist, angeordnet ist.

11. System nach Anspruch 10, wobei eine Gesamtwiderstandsnetzwerkkombination des ersten Widerstands, des zweiten Widerstands und des dritten Widerstands ein Maximum von 1000 bis 1200 Ohm aufweist.

12. System nach Anspruch 1, ferner umfassend eine externe Rückführungselektrode, die elektrisch mit dem Signalgenerator koppelbar ist, und wobei der Katheter die gepulste elektrische Feldenergie in monopolarer Weise mit der externen Rückführungselektrode abgibt.

13. System nach Anspruch 1, wobei der Behandlungskatheter mindestens einen Kontakt aufweist, wobei das System ferner einen Schnittstellenverbinder umfasst, der den mindestens einen Kontakt sowohl mit dem Generator als auch mit einem elektroanatomischen Abbildungssystem elektrisch koppelt, wobei der Schnittstellenverbinder so konfiguriert ist, dass die Elektrode, die zum Abgeben gepulster elektrischer Feldenergie ausgewählt ist, nicht gleichzeitig sowohl mit dem Generator als auch mit dem elektroanatomischen Abbildungssystem elektrisch verbunden ist.

14. System nach Anspruch 13, wobei der Schnittstellenverbinder ein Schaltsystem beinhaltet, das einen ersten Pfad aus mindestens einem leitenden Draht zwischen der Elektrode, die zum Abgeben gepulster elektrischer Feldenergie ausgewählt ist, und dem Generator und einen zweiten Pfad aus mindestens einem leitenden Draht zwischen der Elektrode, die zum Abgeben gepulster elektrischer Feldenergie ausgewählt ist, und dem elektroanatomischen Abbildungssystem umfasst, wobei das Schaltsystem die Energieübertragung zwischen dem ersten Pfad und dem zweiten Pfad umschaltet.

**Revendications**

1. Système de création d'une lésion dans une zone du tissu cardiaque d'un patient comprenant :

   un cathéter de traitement comprenant un corps de cathéter avec une partie distale et une partie proximale, la partie distale du corps de cathéter comprenant des fils conducteurs menant à une pluralité d'électrodes qui sont électriquement isolées les unes des autres ;
   un générateur couplé électriquement au cathéter de traitement et à la pluralité d'électrodes du cathéter, dans lequel le générateur comprend au moins un algorithme de fourniture d'énergie configuré pour fournir un signal électrique d'énergie de champ électrique pulsé délivré par le cathéter à une ou plusieurs électrodes sélectionnées de manière à créer la lésion dans le tissu cardiaque ; et
   un réseau de composants couplé électriquement entre le cathéter et le générateur, le réseau de composants étant configuré pour maintenir une différence de potentiel entre une ou plusieurs électrodes du cathéter sélectionnées pour délivrer une énergie de champ électrique pulsé et une ou plusieurs autres électrodes du cathéter non sélectionnées pour délivrer une énergie de champ électrique pulsé, en dessous d'une différence de potentiel seuil qui empêche la formation d'un arc ou d'un court-circuit entre une ou plusieurs paires des fils électroconducteurs ou paires de la pluralité d'électrodes.

2. Système selon la revendication 1, dans lequel l'énergie de champ électrique pulsé comprend une énergie de champ électrique pulsé ayant des impulsions biphasiques dans une pluralité de paquets.

3. Système selon la revendication 1 ou 2, dans lequel l'énergie de champ électrique pulsé a une tension d'au moins 2000V.

4. Système selon la revendication 1, dans lequel le cathéter est configuré pour éviter la formation d'un arc électrique entre une ou plusieurs paires des fils électroconducteurs lorsqu'il reçoit une énergie de champ électrique pulsé ayant une tension allant jusqu'à un niveau de tension prédéterminé et dans lequel l'énergie de champ électrique pulsé délivrée par le générateur de signaux comprend une énergie supérieure au niveau de tension prédéterminé, le réseau de composants empêchant la formation d'un arc électrique entre une ou plusieurs paires des fils électroconducteurs ou paires de la pluralité d'électrodes.

5. Système selon la revendication 4, dans lequel le niveau de tension prédéterminé est compris entre 1000 et 1500 volts.

6. Système selon l'une quelconque des revendications 1 à 5, dans lequel le cathéter comprend un cathéter d'ablation par radiofréquence.

7. Système selon la revendication 1, dans lequel le réseau de composants comprend au moins une résistance, un inducteur ou une diode.

8. Système selon la revendication 1, dans lequel le réseau de composants comprend une pluralité de résistances.

9. Système selon la revendication 8, dans lequel chacune de la pluralité de résistances est disposée entre un fil électroconducteur couplé électriquement à une électrode sélectionnée pour délivrer une énergie de champ électrique pulsé et un fil électroconducteur couplé électriquement à une électrode qui n'est pas sélectionnée pour délivrer une énergie de champ électrique pulsé.

10. Système selon la revendication 8, dans lequel la pluralité d'électrodes comprend une électrode sélectionnée pour délivrer l'énergie de champ électrique pulsé et trois électrodes non sélectionnées pour délivrer l'énergie de champ électrique pulsé, et dans lequel la pluralité de résistances comprend

   une première résistance disposée entre un fil électroconducteur de livraison couplé électriquement à l'électrode sélectionnée pour délivrer l'énergie de champ électrique pulsé et un premier fil électroconducteur couplé électriquement à une première des trois électrodes non sélectionnées pour délivrer l'énergie de champ électrique pulsé,

une deuxième résistance disposée entre le fil électroconducteur de livraison couplé électriquement à l'électrode sélectionnée pour délivrer l'énergie de champ électrique pulsé et un deuxième fil électroconducteur couplé électriquement à une deuxième des trois électrodes non sélectionnées pour délivrer l'énergie de champ électrique pulsé, et

une troisième résistance disposée entre le fil électroconducteur de livraison couplé électriquement à l'électrode sélectionnée pour délivrer l'énergie de champ électrique pulsé et un troisième fil électroconducteur couplé électriquement à une troisième des trois électrodes non sélectionnées pour délivrer l'énergie de champ électrique pulsé.

11. Système selon la revendication 10, dans lequel une combinaison de réseau de résistance totale de la première résistance, de la deuxième résistance et de la troisième résistance a un maximum de 1000 à 1200 ohms.

12. Système selon la revendication 1, comprenant en outre une électrode de retour externe couplée électriquement au générateur de signaux et dans lequel le cathéter délivre l'énergie de champ électrique pulsé de manière monopolaire avec l'électrode de retour externe.

13. Système selon la revendication 1, dans lequel le cathéter de traitement a au moins un contact, le système comprenant en outre un connecteur d'interface qui relie électriquement l'au moins un contact au générateur et à un système de cartographie électroanatomique, dans lequel le connecteur d'interface est configuré de telle sorte que l'électrode sélectionnée pour délivrer une énergie de champ électrique pulsé n'est pas connectée électriquement au générateur et au système de cartographie électroanatomique simultanément.

14. Système selon la revendication 13, dans lequel le connecteur d'interface comprend un système de commutation comprenant un premier chemin d'au moins un fil conducteur entre l'électrode sélectionnée pour délivrer une énergie de champ électrique pulsé et le générateur et un second chemin d'au moins un fil conducteur entre l'électrode sélectionnée pour délivrer une énergie de champ électrique pulsé et le système de cartographie électroanatomique, dans lequel le système de commutation fait basculer la transmission d'énergie entre le premier chemin et le second chemin.

FIG-1

EP 4 076 240 B1

FIG. 2A

FIG. 2B

EP 4 076 240 B1

FIG_4

FIG_3

EP 4 076 240 B1

FIG. 6

EP 4 076 240 B1

FIG. 6

40

Pulse Voltage Asymmetry : Ex 2500-1250-2500 V

FIG. 7

Pulse Length Asymmetry : Ex 830-415-830ns

FIG. 8

EP 4 076 240 B1

400

402    404

430    432

Pulse
width

416    412    406

418

FIG. 9

Pulse Asymmetry (pulse length and/or pulse voltage): Ex 2500V@830ns - 0 - 2500V@830ns
*Note: Because no opposing polarity, there is no appreciable difference when considering $t_s$ v. $t_d$*

Symmetric, pure
$t_s = t_d = 0$

Symmetric
$t_s = t_d \neq 0$

Asymmetric
$t_s < t_d \neq 0$

Asymmetric
$t_s > t_d \neq 0$

1 pulse  430

430

430

430

Voltage

time

1 cycle

1 cycle

1 cycle

1 cycle

402

404

405

407

FIG. 10

Pulse Voltage and Timing Variable Asymmetry : Multiple combinations and permutations possible

408  430  430

408  430  408  430

408  430  408  430

408  430  408  430

Voltage

410

410

410

410

410

410

410

410

410

1 cycle

1 cycle

1 cycle

1 cycle

402

404

405

407

FIG. 11

FIG. 12

FIG. 13

FIG. 14A

FIG. 14B

FIG. 15

E2,E3 & E4 Uncovered

FIG. 16A

E2,E3 & E4 Uncovered & Resistors

FIG. 16B

FIG. 17A

FIG. 17B

FIG. 17C

FIG-18

FIG-19

## Current Density Distribution

FIG_20

FIG_21

FIG_22

Force vs Lesion 28A, 1.4 ms

Force vs Lesion 35A, 1.6 ms

FIG_23A

FIG_23B

High Dose, No Irrigation, 119 bpm

FIG_24

FIG. 25

FIG. 26

FIG. 27

FIG. 28

FIG. 29

FIG. 30

FIG. 31

FIG. 32

FIG. 33

EP 4 076 240 B1

FIG. 34

FIG. 35

FIG. 36

FIG. 37A

FIG. 37B

FIG. 38A

FIG. 38B

FIG. 39

FIG. 40A

FIG. 40B

FIG. 41

FIG. 42

FIG_43A

FIG_43B

FIG_43C

FIG_43D

FIG_43E

FIG_44A

FIG_44B

FIG_45

FIG. 46A

FIG. 47.

FIG. 46B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20190201089 A
- WO 2019133606 A
- WO 2019133608 A
- US 2018067504 W
- US 62610430 B
- US 62693622